# EUROPÄISCHE PATENTSCHRIFT

(11) **EP 2 978 402 B1**
(45) Veröffentlichungstag und Bekanntmachung des Hinweises auf die Patenterteilung: **16.10.2019**
(21) Anmeldenummer: 14715870.3
(22) Anmeldetag: 27.03.2014
(51) Int. Cl.: A61K 8/49, C07K 14/78

(54) **STABILISIERTES POLYRIBONUCLEOTID CODIEREND FÜR EIN ELASTISCHES FASERPROTEIN**
STABILIZED POLYRIBONUCLEOTIDE CODING FOR AN ELASTIC FIBROUS PROTEIN
POLYRIBONUCLÉOTIDE STABILISÉ CODANT POUR UNE PROTÉINE FIBREUSE ÉLASTIQUE

(30) Priorität: 28.03.2013 DE 102013005361
(43) Veröffentlichungstag der Anmeldung: 03.02.2016
(73) Patentinhaber: Eberhard Karls Universität Tübingen Medizinische Fakultät, 72074 Tübingen (DE)
(72) Erfinder: WENDEL, Hans-Peter, 72336 Balingen (DE); KELLER, Timea, 72072 Tuebingen (DE); NOLTE, Andrea, 71106 Magstadt (DE); AVCI-ADALI, Meltem, 72138 Kirchentellinsfurt (DE); WALKER, Tobias, 72074 Tuebingen (DE)
(74) Vertreter: Witte, Weller & Partner Patentanwälte mbB
(86) Internationale Anmeldenummer: PCT/EP2014/056233
(87) Internationale Veröffentlichungsnummer: WO 2014/154844

(56) Entgegenhaltungen:
- WO-A1-2009/127230
- WO-A1-2011/008904
- WO-A2-2007/024708
- WO-A2-2008/052770
- WO-A2-2008/100789
- WO-A2-2011/012316
- XIONG J ET AL: "Elastic fibers reconstructed using adenovirus-mediated expression of tropoelastin and tested in the elastase model of abdominal aortic aneurysm in rats", JOURNAL OF VASCULAR SURGERY, C.V. MOSBY CO., ST. LOUIS, MO, US, Bd. 48, Nr. 4, 1. Oktober 2008 (2008-10-01), Seiten 965-973, XP025668638, ISSN: 0741-5214, DOI: 10.1016/J.JVS.2008.04.016 [gefunden am 2008-06-30]

## Beschreibung

Die vorliegende Erfindung betrifft ein Polyribonucleotid, eine kosmetische und pharmazeutische Zusammensetzung, die das Polyribonucleotid aufweist und ein Medizinprodukt, das das Polyribonucleotid bzw. die Zusammensetzung aufweist.

Elastische Fasern sind die größten Strukturen der extrazellulären Matrix. Sie verleihen dem Gewebe elastische Eigenschaften. Die elastischen Fasern bestehen aus zwei morphologisch distinkten Komponenten. Die erste und größte Komponente der reifen Faser ist das Elastin. Die zweite Komponente sind die Mikrofibrillen, welche hauptsächlich aus Fibrillin bestehen und mit weiteren Proteinen, wie den Mikrofibrillenassoziierten Glycoproteinen (MAGPs), Fibulinen und dem Elastin-Mikrofibrillen-Interfacelokalisierten Protein (EMILIN), assoziiert sind. In die Quervernetzung der elastischen Fasern ist die Lysyloxidase (LOX) involviert.

Elastin und dessen löslicher Vorläufer Tropoelastin gehören zu den Hauptstrukturproteinen des Körpers. Es verleiht dem Bindegewebe der Haut Form und Halt und ist für die Dehnbarkeit von Arterien und der Lunge verantwortlich.

Elastin wird vom *ELN*-Gen codiert. Mutationen im *ELN*-Gen können erbliche Erkrankungen verursachen wie Dermatochalasis, Williams-Beuren-Syndrom und subvalvuläre angeborene Aortenstenose (SVAS).

Arteriosklerotische Blutgefäße unterliegen einem Elastinverlust, der durch die an der Regeneration beteiligten Zellen nicht auf natürlichem Wege ausgeglichen werden kann. Dies ist darin begründet, dass Elastin-bildende Zellen nur bis zu einem bestimmten Alter und während des Wachstums des Organismus neues Elastin synthetisieren und sezernieren. Das Protein wird anschließend im Extrazellularraum untereinander und mit anderen Proteinen des Bindegewebes quervernetzt. Da Elastin durch die Vernetzung besonders langlebig ist, ist der Bedarf eines Organismus nach Erreichen der vollen Körpergröße gedeckt und die Synthese wird weitgehend eingestellt.

Durch die verminderte Elastinsynthese im Alter kommt es zur Erschlaffung der Haut und zur Faltenbildung.

Zufriedenstellende oder gar ursächliche Therapien einer defizitären Synthese von elastischem Faserprotein, insbesondere Elastin, stehen nicht zur Verfügung.

Hirano et al. (2007), Functional rescue of elastin insufficiency in mice by the human elastin gene: implications for mouse models of human disease, Circulation Research 101: 523-531, beschreiben die Einschleusung von humanem Elastin mittels eines DNA-Plasmids in Mäuseoozyten. Für eine therapeutische Anwendung im Menschen ist dieser Ansatz jedoch nicht geeignet.

Die WO 2007/024708 beschäftigt sich allgemein mit der Stabilisierung von mRNA und der Reduzierung ihrer Immunogenität durch Einführung von modifizierten Nukleotiden. Die WO 2009/127230 befasst sich mit der Verwendung von nukleotidmodifizierter mRNA als Bestandteil einer immunsuppressiven Zusammensetzung. In der WO 2008/052770 wird eine basenmodifizierte mRNA beschrieben. Die WO 2011/008904 schlägt die Verwendung einer Elastin-cDNA zur kosmetischen Behandlung vor. Xiong et al. (2008), J. Vas. Surg., Bd. 48, S. 965-973 beschreibt die AV-vermittelte Expression von Tropoelastin in einem Elastase-Rattenmodell für abdominale Aorten-Aneurysmen.

Vor diesem Hintergrund ist es eine Aufgabe der vorliegenden Erfindung eine Substanz bereitzustellen, mit der die eingangs genannten Probleme gelöst werden können. Insbesondere soll eine solche Substanz bereitgestellt werden, mit der einem Mangel von elastischen Fasern bzw. elastischem Faserprotein entgegenwirkt und die Synthese von elastischen Fasern bzw. elastischem Faserprotein stimuliert werden kann.

Diese Aufgabe wird durch die Bereitstellung eines Polyribonucleotids codierend für ein Elastin/Tropoelastin gelöst, das eine Nucleotidsequenz aufweist, die zumindest eine das Polyribonucleotid stabilisierende chemische Modifizierung aufweist, wobei zumindest ca. 25 % der Uridine des Polyribonucleotid durch Pseudouridine ersetzt wird und ca. 25 % der Cytidine des Polyribonucleotids durch 5-Methylcytidine ersetzt wird, um das Polyribonucleotid zu stabilisieren.

Die Erfinder haben überraschenderweise festgestellt, dass einer Defizienz in der Synthese von elastischem Faserprotein ursächlich entgegengewirkt werden kann, indem der Zelle die Codierungssequenz in direkt translatierbarer Form bereitgestellt wird. Das erfindungsgemäße Polyribonucleotid kann in die zu regenerierenden Zellen eingebracht werden und *in situ* die Synthese von Elastin/Tropoelastin induzieren. Die Zellen schleusen das synthetisierte Elastin/Tropoelastin in den natürlichen Pfad der Assemblierung der elastischen Fasern ein. Dadurch wird nicht nur die Synthese des Elastin/Tropoelastin induziert sondern auch die Neusynthese der elastischen Fasern. Weder die Applikation des elastischen Proteins als solchem noch die anderer Proteine, die in die Genese von elastischen Fasern involviert sind, konnten hier bislang vergleichbare Ergebnisse liefern.

Die Stabilisierung von Polyribonucleotiden, bspw. von mRNA, ist im Stand der Technik umfangreich beschrieben. Verwiesen wird auf die folgenden Publikationen: US 2009/0093433, WO 2011/012316, WO 2012/135805, WO 2012/045082, WO 2012/019168, WO 2012/045075, WO 2012/158736. Ferner wird die Stabilisierung von mRNA durch chemische Modifizierung von Ribonucleotiden in den Publikationen von Warren et al. (2010), High efficient reprogramming to pluripotency and directed differentiation of human cells with synthetic modified mRNA, Cell Stem Cell 7, S. 618 bis 630, und Kormann et al. (2011), Expression of therapeutic proteins after delivery of chemically modified mRNA in mice, Nature Biotechnology, Vol. 29, Nr. 2, S. 154 bis 159, Mandal und Rossi (2013), Reprogramming human fibroblast to pluripotency using modified mRNA, Nature Protocols, Vol. 8, Nr. 3, S. 568 bis 582, beschrieben.

Verfahren zur Synthese von Polyribonucleotiden, bspw. von mRNA, sind im Stand der Technik ebenfalls umfangreich beschrieben. Eines der geeigneten Verfahren ist die In-vitro-Transkription (IVT). Die resultierende mRNA wird auch als IVT-mRNA bezeichnet.

Die Erfinder haben erkannt, dass die Synthese von elastischem Faserprotein *in situ*, d.h. in der Zelle, eine Defizienz bspw. aufgrund einer Mutation oder aber aufgrund einer altersbedingten Einstellung der natürlichen Synthese, zielgerichtet ausgeglichen werden kann.

Durch die chemische Modifizierung ist das Polyribonucleotid ausreichend stabil, um von der zelleigenen Maschinerie translatiert zu werden. Das erfindungsgemäße Polyribonucleotid ist jedoch hinreichend instabil, um lediglich temporäre Wirkung zu entfalten, so dass Nebenwirkungen weitgehend vermieden werden.

Wie die Erfinder zeigen konnten, fällt die Immunantwort eines mit dem erfindungsgemäßen Polynucleotid behandelten Organismus deutlich geringer aus als bei Verwendung eines chemisch nicht modifizierten Referenzpolyribonucleotids. Dadurch wird der therapeutische Nutzen des erfindungsgemäßen Polyribonucleotids zusätzlich erhöht.

Die der Erfindung zugrundeliegende Aufgabe wird damit vollkommen gelöst.

Erfindungsgemäß ist das elastische Faserprotein Elastin/Tropoelastin.

Diese Maßnahme hat den Vorteil, dass das erfindungsgemäße Polyribonucleotid zur Induktion der *In*-*situ*-Synthese eines wichtigen elastischen Faserproteins ausgestaltet ist. Bevorzugt handelt es sich bei dem genannten elastischen Faserprotein um die humane Variante, so dass die Verwendung im Menschen begünstigt wird.

Erfindungsgemäß ist es ferner bevorzugt, wenn das Polyribonucleotid eine mRNA ist.

Diese Maßnahme hat den Vorteil, dass das Polyribonucleotid in einer Form bereitgestellt wird, die von der zelleigenen Proteinsynthesemaschinerie unmittelbar verwertet werden kann. Dabei kann es sich bspw. um eine *in vitro* transkribierte mRNA (IVT-mRNA) handeln.

Erfindungsgemäß ist insbesondere eine durch chemische Modifizierung stabilisierte mRNA des humanen Elastins, Transkriptvariante 1 (cDNA, NCBI-Datenbank NM_000501.2; SEQ ID Nr. 2), Transkriptvariante 2 (cDNA, NCBI-Datenbank NM_001081752.1; SEQ ID Nr. 3), Transkriptvariante 3 (cDNA; NCBI-Datenbank NM_001081753.1; SEQ ID Nr. 4), Transkriptvariante 4 (cDNA, NCBI-Datenbank NM_001081754.1; SEQ ID Nr. 5), Transkriptvariante 5 (cDNA, NCBI-Datenbank NM_001081755.1; SEQ ID Nr. 6) bevorzugt.

Die zumindest eine das Polyribonucleotid stabilisierende chemische Modifizierung umfasst ein modifiziertes Uridin und/oder Cytidin.

Durch diese Maßnahme haben die Erfinder sich die bspw. von Kormann et al. (a.a.O.) beschriebene Erkenntnis zunutze gemacht, dass die mRNA nach chemischer Modifizierung der Uridinribonucleotide und/oder Cytidinribonucleotide von Strukturen des immunsystems, wie den signaltransduktionsvermittelnden PRRs ("pattern recognition receptors") oder "Toll-ähnlichen Rezeptoren", schlechter erkannt werden, somit eine deutlich abgeschwächte Immunantwort auslösen und eine längere Halbwertszeit erlangen.

Erfindungsgemäß umfasst ein Nucleosid auch das entsprechende Nucleotid, das gegenüber dem Nucleosid zusätzlich Phosphatreste aufweist.

Das erfindungsgemäß chemisch modifizierte Uridin ist Pseudouridin.

Das erfindungsgemäß chemisch modifizierte Cytidin ist 5-Methylcytidin.

Erfindungsgemäß werden zumindest 25 % der Uridine und/oder Cytidine modifiziert.

Obgleich auch zumindest ca. 50 % bzw. ca. 100 % der Uridine und/oder Cytidine modifiziert sein können, haben die Erfinder festgestellt, dass eine Modifizierung von bis zu ca. 25 % der Nucleoside für eine Stabilisierung des erfindungsgemäßen Polyribonucleotids und Reduzierung der Immunantwort ausreichend ist. Dies hat den Vorteil, dass die Kosten für die Herstellung des erfindungsgemäßen Polyribonucleotids deutlich niedriger als bei einer 100 %igen Modifizierung ausfallen.

Nach einer bevorzugten Weiterbildung ist die chemische Modifizierung ausgewählt aus der Gruppe bestehend aus: 5'-Cap-Struktur, vorzugsweise eine 5'-Guanin-Kappe, Poly-(A)-Schwanz, ein Kappenstrukturanalog [anti-reverse cap analog (ARCA; 3'O-Me-m⁷G(5')ppp(5')ppp(5')G)], eine Verstärkung der Translations-Initiations-Sequenz beim Startcodon AUG, z.B. durch die Sequenz (CCCCGC)aucGagAUG.

Durch diese Maßnahme wird auf vorteilhafte Art und Weise eine zusätzliche Stabilisierung des erfindungsgemäßen Polyribonucleotids erzielt.

Nach einer bevorzugten Weiterbildung weist das erfindungsgemäße Polyribonucleotid die Sequenz SEQ ID Nr. 1 auf, bei der zumindest ca. 25 % der Uridine, und/oder bei der zumindest ca. 25 % der Cytidine chemisch modifiziert sind.

Wie oben erwähnt, können zwar auch zumindest ca. 50 % bzw. zumindest ca. 100 % der Nucleoside modifiziert sein, eine ca. 25 %ige Modifizierung ist jedoch ausreichend.

Die weiter oben angegebene Definition für "chemisch modifiziert" gilt hier entsprechend. Vorzugsweise erfolgt ein Austausch von Uridin (U) durch Pseudouridin bzw. Pseudouridintriphosphat (ΨUTP) und/oder von Cytidin (C) durch 5-Methylcytidin bzw. 5-Methylcytidintriphosphat (mCTP).

Das erfindungsgemäße Polyribonucleotid codiert vorzugsweise für eine Aminosäuresequenz, die ausgewählt ist aus der Gruppe bestehend aus: SEQ ID Nr. 7, SEQ ID Nr. 8, SEQ ID Nr. 9, SEQ ID Nr. 10, und SEQ ID Nr. 11.

Diese Maßnahme hat den Vorteil, dass das erfindungsgemäße Polynucleotid für eine der verschiedenen Isoformen des Elastins (SEQ ID Nr. 7: Isoform a, NCBI-Datenbank NP_00049.2; SEQ ID Nr. 8: Isoform b, NCBI-Datenbank NP_001075221.1; SEQ ID Nr. 9: Isoform c, NCBI-Datenbank NP_001075222.1; SEQ ID Nr. 10: Isoform d, NCBI-Datenbank NP_001075223.1; SEQ ID Nr. 11: Isoform e, NCBI-Datenbank NP_001075224.1) codiert. Die Elastin-Isoformen weisen die erfindungsgemäß erkannte Wirkung auf.

Erfindungsgemäß ist es bevorzugt, wenn das Polyribonucleotid zur Induktion der Synthese von elastischen Faserproteinen, insbesondere bei altersbedingtem Elastizitätsverlust der Haut (Faltenbildung), Förderung der Wundheilung, zur Behandlung einer defizitären Synthese von elastischem Faserprotein, bzw. zur Behandlung einer Krankheit ausgebildet ist, die ausgewählt ist aus der Gruppe bestehend aus: Arteriosklerose, Aortenstenose, Aortenaneurysma, Lungenemphysem, Dermatochalasis, Williams-Beuren-Syndrom, subvalvuläre angeborene Aortenstenose (SVAS).

Mittels des erfindungsgemäßen Polyribonucleotids kann ferner das Vaginalgewebe behandelt werde, bspw. in Folge einer Schwangerschaft, zur Stiumulation der Elastinsynthese und Wiedererlangung der Elastizität.

Ferner kann das erfindungsgemäße Polyribonucleotid ausgebildet sein für dentale Anwendungen, bspw. zur Rekonstruktion und/oder Regeneration von Weich- und Hartgeweben des Periodontiums. Das erfindungsgemäße Polyribonucleotid ist deshalb bevorzugt zur transdermalen Applikation vorgesehen oder ausgebildet.

Diese Maßnahmen haben den Vorteil, dass das erfindungsgemäße Polyribonucleotid zur Behandlung wichtiger Erkrankungen und Erscheinungen ausgebildet ist, die infolge einer reduzierten Synthese von elastischem Faserprotein entstehen können.

Hierin beschrieben ist demnach auch die Verwendung des erfindungsgemäßen Polyribonucleotid zu den vorstehend genannten Zwecken.

Ferner kann die Erfindung in einem Verfahren zur Induktion der Synthese von elastischen Faserproteinen eingesetzt werden, insbesondere zu den vorstehend genannten Zwecken, das folgende Schritte aufweist: (1) Bereitstellung des erfindungsgemäßen Polyribonucleotids, ggf. in einer pharmazeutisch/kosmetisch akzeptablen Formulierung, und (2) Verabbreichung des Polyribonucleotids in oder an einen Organismus.

Die Verabreichung des Polyribonucleotids in oder an einen Organismus kann über eine topische Applikation erfolgen, bspw. auf die Haut des Organismus, vorzugsweise die menschliche Haut. Dazu kann das Polynucleotid Bestandteil einer dermatologischen Darreichungsform wie einer Creme, Lotion, Salbe etc. sein. Die Verabreichung kann aber auch über geeignete Darreichungsformen systemisch, oral, intravenös, intraarteriell, intramuskulär, intrathekal, subkutan, intraperitoneal, intrakardial, intravitreal, oder intraossär etc. erfolgen.

Die transdermale Verabreichung des erfindungsgemäßen Polynucleotids kann mittels Mikronadeln, Nanopatches, Nanopartikeln oder mittels einer Genkanone erfolgen. Ferner sind aktive Systeme geeignet, die sich der Iontophorese bedienen. Dabei wird ein sehr geringer elektrischer Strom durch die Haut geleitet, der geladene Moleküle transportiert. Ein Beispiel hierfür ist das lontophorese-LTS-TTS-System der Firma LTS Lohmann Therapie-Systeme AG, Andernach, Deutschland.

Vor diesem Hintergrund betrifft die vorliegende Erfindung auch eine Zusammensetzung, die das erfindungsgemäße Polyribonucleotid aufweist. Bei der erfindungsgemäßen Zusammensetzung kann es sich um eine kosmetische und/oder pharmazeutische Zusammensetzung handeln, die eine kosmetisch bzw. pharmazeutisch akzeptable Formulierung aufweist. Kosmetisch und pharmazeutisch akzeptable Formulierungen sind im Stand der Technik allgemein bekannt. Sie werden bspw. in der Abhandlung von Kibbe et al., Handbook of Pharmaceutical Excipients, 5. Auflage (2006), *American Pharmaceutical Association*, beschrieben. Die Zusammensetzungen können als Monopräparat ausgebildet sein, die als alleinigen Wirkstoff das erfindungsgemäße Polyribonucleotid enthalten. Sie können aber auch Zusätze und ggf. weitere Wirk- und Hilfsstoffe enthalten, die für die erfindungsgemäßen Verwendungen vorteilhaft sind, worunter Transfektionsmittel wie Liposomen, Hydrogele, kationische Polymere oder Peptide fallen, Salze, Bindemittel, Lösungsmittel, Dispersionsmittel und weitere in Zusammenhang mit der Formulierung von Kosmetika und Arzneimitteln üblicherweise verwendete Stoffe fallen.

Nach einer erfindungsgemäß bevorzugten Weiterentwicklung kann die Zusammensetzung zusätzlich ein Immunsuppressivum, vorzugsweise einen Interferon-Inhibitor aufweisen.

Diese Maßnahme hat den Vorteil, dass die durch die chemische Modifizierung bereits reduzierte Immunantwort eines mit dem erfindungsgemäßen Polyribonucleotid behandelten Wirtes weiter reduziert wird. Die Unterdrückung der Immunantwort nach Verabreichung einer therapeutischen mRNA durch Verwendung des Interferon-Inhibitors B18R, der zur erfindungsgemäßen Verwendung geeignet ist, ist im Stand der Technik dokumentiert, bspw. in *Warren et al.* (a.a.O.).

Ein weiterer Gegenstand der vorliegenden Erfindung betrifft ein Medizinprodukt, bspw. ein Patch oder Implantat, das das erfindungsgemäße Polyribonucleotid bzw. die erfindungsgemäße Zusammensetzung aufweist bzw. mit Letzteren beschichtet ist. Bei dem Implantat kann es sich um ein medizinisches Implantat, vorzugsweise ein Stent einschließlich eines Koronarstents, oder Gefäßimplantat, Stentgraft oder ein Knochenimplantat handeln.

Über das Implantat lassen sich zielgerichtet arteriosklerotische Blutgefäße und/oder lokale Gewebeareale, wie Vaginalgewebe, Weich- und Hartgewebe des Priodontiums zur Wiedererlangung der Elastizität behandeln.

Ein weiterer Gegenstand der vorliegenden Erfindung betrifft eine Wundauflage, die mit dem erfindungsgemäßen Polyribonucleotid beschichtet ist.

Eine solche Wundauflage kann durch Induktion der Elastinsynthese die Narbenbildung reduzieren und die Elastizität des Narbengewebes erhalten.

Die vorliegende Erfindung wird nun anhand von Ausführungsbeispielen näher erläutert, aus denen sich weitere Eigenschaften und Vorteile der Erfindung ergeben. Die Beispiele sind rein illustrativ und schränken die Reichweite der Erfindung nicht ein. Dabei wird Bezug genommen auf die beiliegenden Figuren. Diese zeigen:

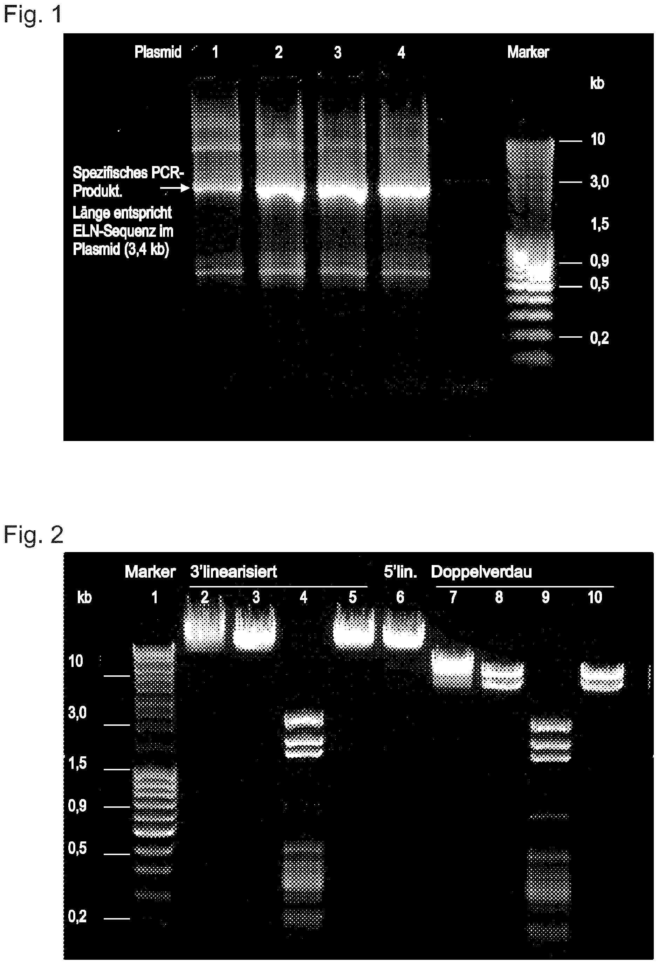

- Fig. 1:: Plasmidpräparationen aus vier einzelnen selektierten Bakterienkolonien wurden anhand ELN-spezifischer PCR auf das Insert überprüft. Nachweise der PCR-Produkte mittels Agarose-Gelelektrophorese, 1,2 % (120 V, 30 min). PCR-Zyklen: Denaturieren 3 min, 94 °C, 1. 40 sec, 94 °C - 2. 1 min, 58 °C - 3. 2,5 min, 72 °C; finale Amplifikation: 7 min, 72 °C;
- Fig. 2:: Eindeutig schneidende Restriktionsenzyme wurden anhand von Testverdauen des Plasmids und anschließender Analyse auf 1%igem Agarose-Gel (120 V, 40 min) ermittelt. Banden: 1-Marker/DNA-Leiter, 2-5: Testverdau des Plasmids mit verschiedenen stromabwärts (3') schneidenden Restriktionsenzymen: 2: Not I, 3: Xba I, 4: Bfa I, 5: Xho I, 6: Verdau mit stromaufwärts (5') schneidendem Enzym, EcoRI, 7-10: stromabwärts schneidende Enzyme wurden mit Ecor I kombiniert (Doppelverdau), Reihenfolge wie 2-5. kb = Kilobasen;

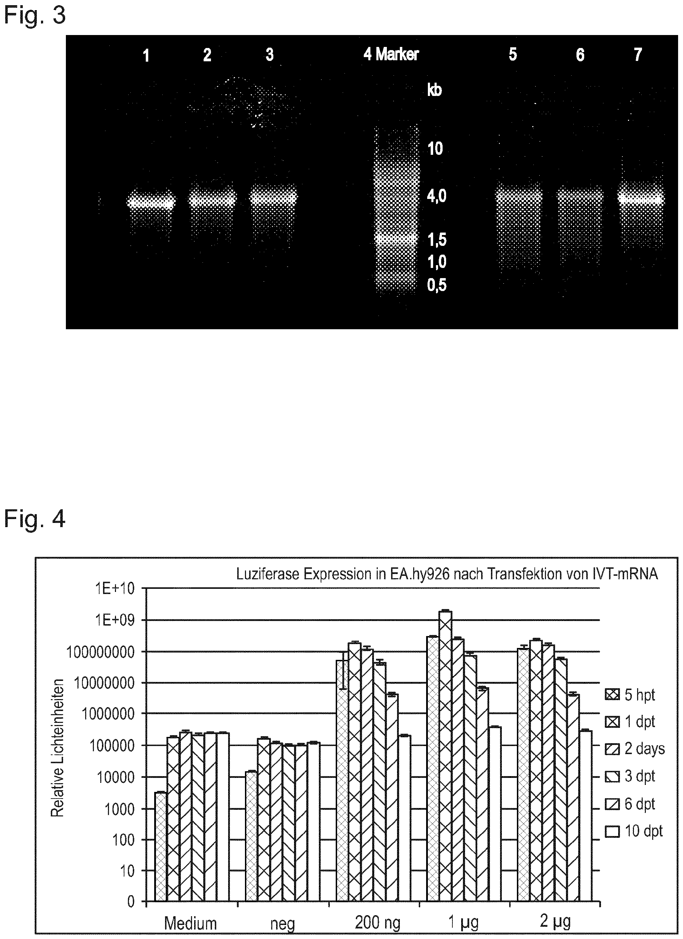

- Fig. 3:: In-vitro synthetisierte ELN-mRNA in denaturierender Agarose-Gelelektrophorese 1 % (16 % Formaldehyd, 100 V, 45 min). 1: IVT-Reaktion nach 3 h Inkubation; 2: IVT-Reaktion mit Polyadenylisierungsmix und 25 min Inkubation; 3; wie 2, nach 45 min; 4: Marker; 5-6: Aufgereinigte, polyadenylierte ELN-mRNAs; 5, 6: mit Ambion MEGAclear Kit (Katalognummer AM1908); 7: mit Qiagen RNeasy Midi Kit, doppelte RNA-Menge (Katalognummer: 75142);
- Fig. 4:: Nachweis Reportergenexpression: Detektion der Luziferaseexpression nach Transfektion von IVT-Luziferase-mRNA in EA.hy926. hpt = Stunden nach Transfektion; dpt = Tage nach Transfektion;

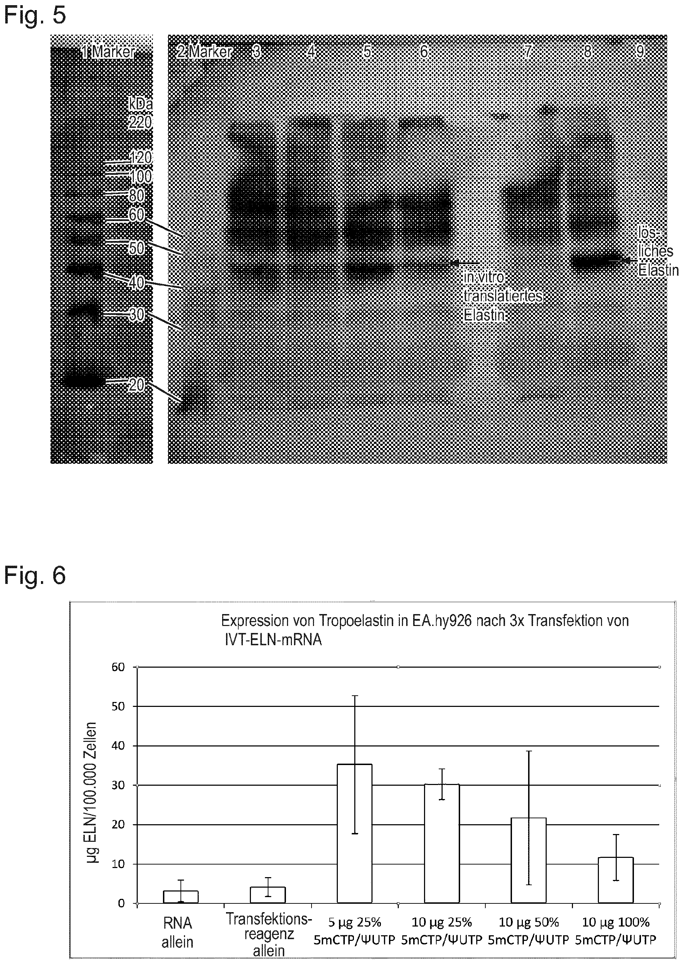

- Fig. 5:: In-vitro synthetisiertes ELN-Protein auf Grundlage von vier verschiedenen in-vitro synthetisierten ELN-mRNA und Kontrollen: 1, 2: Marker; 3-6; Proteinsynthese mit ELN-mRNAs aus verschiedenen in vitroTranskriptionsreaktionen (5: mRNA mit modifizierten Nucleotiden); 7: Invitro Translationsreaktion ohne mRNA (Negativkontrolle); 8: wie 7, aber Zugabe von humanem, löslichen Elastin-Protein von Calbiochem (Katalognummer: 324751) nach Inkubationszeit; 9: nur in Wasser gelöstes Elastin-Protein von Calbiochem - laut Datenblatt sollte das Protein als Schmierbande zwischen 5 und 60 kDa laufen. Natives Elastin-Protein wird anhand des verwendeten Antikörpers auf einer Höhe von ca. 50 kDa detektiert.
- Fig. 6:: Nachweis der Elastinexpression durch Messung der Tropoelastinmenge in EA.hy926-Zellen nach Transfektion an drei aufeinanderfolgenden Tagen mit IVT-ELN-mRNA; diese enthielten verschiedene Anteile von modifizierten Nucleinsäuren (mCTP/ΨUTP) und Lipofectamin2000. Als Negativkontrollen dienten RNA oder Lipofectamin2000 allein.

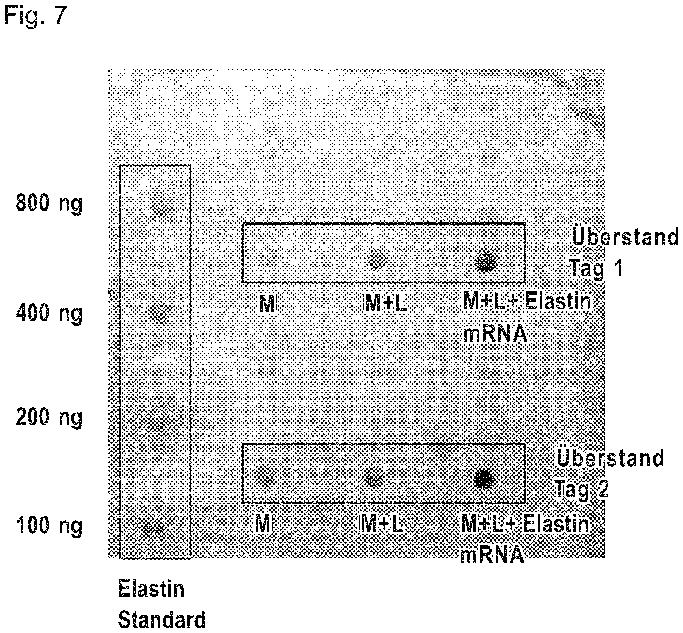

- Fig. 7: Nachweis der Elastinexpression in Überstand der EA.hy926-Zellen nach Transfektion mit 100% modifizierter (mCTP/ΨUTP) IVT-ELN-mRNA mittels Dot-Blot-Assays; M: nur Zellkulturmedium, M+L: Zellkulturmedium plus Transfektionsreagenz.

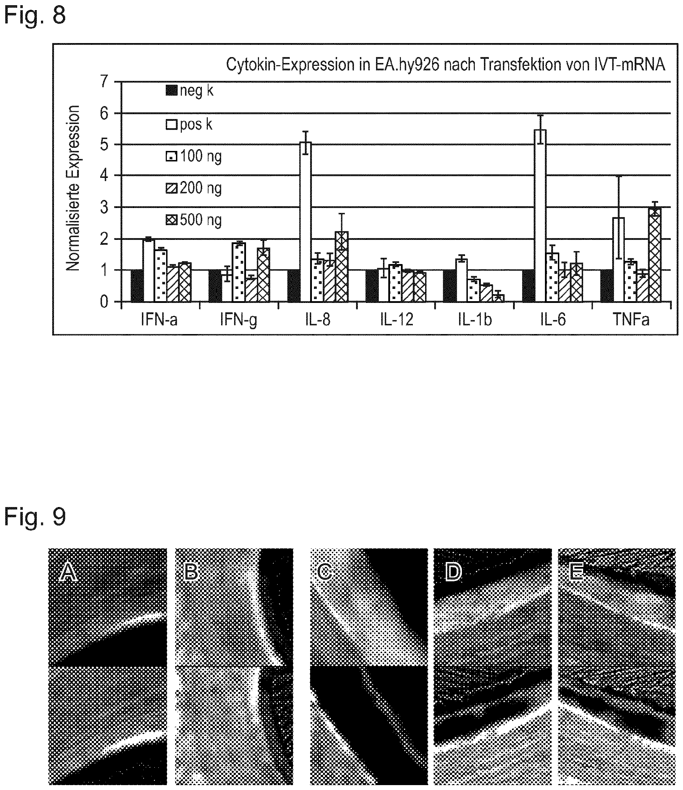

- Fig. 8:: Immunaktivierung von EA.hy926-Zellen nach der Transfektion von IVT-ELN-mRNA; die Expressionen wurden auf das Haushaltsgen GAPDH normalisiert. Die Negativkontrollen wurden auf 1 gesetzt.
- Fig. 9:: Nachweis Reportergenexpression:Mikroskopische Aufnahmen von "gestenteten" Gefäßen, Fokus auf die Grenze zwischen Stent und Gewebe. A, B: negative Kontrollen (unbeschichtete Stents). C-E: eGPF-mRNA-beschichtete Stents. Die Größe der Aufnahmen entspricht einem Bereich von 30 x 30 µm. Obere Abbildungen: Originalaufnahmen. Untere Abbildungen: Aufnahmen mit FIDSAM und Kontrastkorrektur.

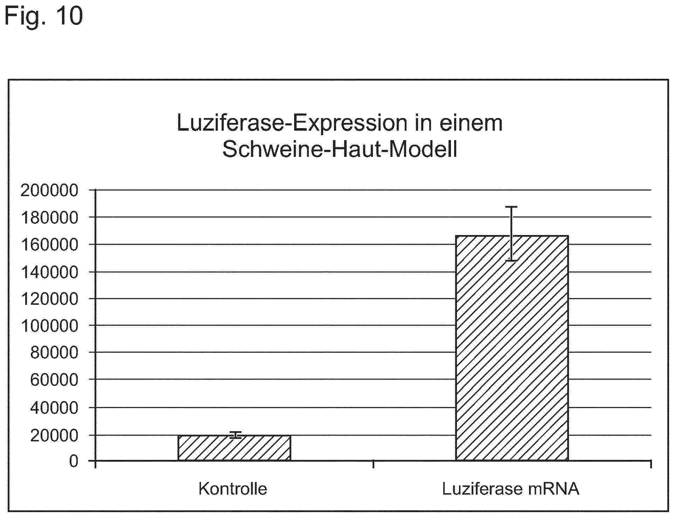

- Fig. 10:: Nachweis der Luziferase-Expression in der Schweinehaut nach Transfektion mit Luziferase-mRNA

### Ausführungsbeispiele

### 1. Plasmidkonstrukte für die RNA-Synthese

Ein Plasmid, das für Elastin, Transkriptvariante 1 (SEQ ID Nr. 2) codierte, und einen Sp6-Promotor enthielt, pCMV-Sp6_ELN, cloniert in *E.coli*, wurde von Thermo Scientific bezogen.

Das Luciferase-codierende, T7-Promotor enthaltende Plasmid, pCMV-GLuc-1, das als Reporter-Gen verwendet wurde, wurde von Nanolight Technology, Inc. bezogen und in EZ-kompetente *E.coli* von Qiagen aus dem Qiagen PCT-Klonierungskit kloniert.

Die Plasmide enthalten Stellen zur Sequenzierung mit Primern, wie bspw. den M13 Vorwärts- und Rückwärtsprimern, und Promotorbereiche für Polymerasen, wie der Sp6 und T7. Diese Sequenzen können 5' oder 3' zu der interessierenden eingefügten Sequenz gefunden werden. Ferner ist der Insertbereich flankiert von kurzen Erkennungssequenzen für spezifische Restriktionsenzyme.

### 2. Verifizierung der Plasmidinserts

Die Plasmide wurden mit dem Qiagen Plasmid Maxi Kit (Qiagen) isoliert.

Insert-spezifische Primer wurden mit einem Designtool für freie Primer von NCBI konstruiert und von Eurofins MWG Operon hergestellt. Die Plasmidinserts wurden mittels PCR mit Insert-spezifischen Primerpaaren verifiziert (Fig. 1), wobei die folgenden Zyklusparameter verwendet wurden: Denaturieren 3 min bei 94 °C; 1. 40 sec bei 94 °C; 2. 1 min bei 58 °C; 3. 2,5 min bei 72 °C; Wiederholen der Schritte 1-3, 28 x; finale Amplifizierung 7 min, 72 °C.

Zusätzlich wurden die Plasmidinserts durch die Firma GATC Biotech mit selbstkonstruierten Insert-spezifischen Primern und mit M13 Vorwärts- und Rückwärtsprimern vollständig sequenziert. Die Sequenzassemblierung erfolgte mit dem DNAbaser-Programm.

### 3. In vitro Synthese von ELN-mRNA mit verschiedenen Anteilen von modifizierten Nucleinsäuren

Für die *in vitro* Transkription (IVT) wurden die Plasmide stromabwärts des interessierenden Gens mit dem Fast Digest Enzyme System (Fermentas/Thermo Scientific) linearisiert und mit dem MiniElute PCR Clean Up Kit (Qiagen) gereinigt. Testverdaue erfolgten vor dem Experiment (Fig. 2).

Die *in vitro* Transkription erfolgte mit dem MEGAscript Sp6 Kit gemäß den Angaben des Herstellers. Für jede 40 µL IVT-Reaktion wurde 1 µg von linearisiertem Template verwendet. Um die Stabilität und Zytokompatibilität von IVT-mRNAs zu optimieren, wurden in den Reaktionen verschiedene Verhältnisse der Nucleinsäuretriphosphate 5-Methylcytidin und Pseudouridin (TriLink Biotechnologies) mit Standardnucleotiden aus dem Kit kombiniert.

**Tab 1: Nucleotidmischungen in den IVT-Reaktionen mit unterschiedlichen Anteilen von 5-Methylcytidin und Pseudouridin**

| | CTP | 5-Methylcytidin | UTP | Pseudouridin |
|---|---|---|---|---|
| 25 % Modifizierung | 5,25 mM | 1,75 mM | 5,25 mM | 1,75 mM |
| 50 % Modifizierung | 3,5 mM | 3,5 mM | 3,5 mM | 3,5 mM |
| 100 % Modifizierung | - | 7 mM | - | 7 mM |

In jeder IVT-Reaktion wurden 6 mM ATP, 3 mM GTP (aus dem Kit) und 2 mM 3'-O-Me-m⁷G(5')ppp(5')G (Anti-Reverse-Kappe-Analog von New England Biolabs) verwendet. Weitere Komponenten wurden hinzugegeben, wie in den Anweisungen zu dem Kit angegeben.

Die Reaktionen wurden für 3,5 Stunden bei 37 °C inkubiert und mit DNase I aus dem Kit am Ende der Inkubationszeit entsprechend den Anweisungen des Herstellers behandelt, um die Template-DNA zu entfernen.

Die Polyadenylierung erfolgte mit dem PolyA Tailing Kit (Ambion) gemäß den Anweisungen des Herstellers.

Die mRNA wurde mit dem RNeasy Mini Kit (Qiagen) gereinigt. Der Nachweis der Elastin-mRNA erfolgte durch denaturierende Agarose-Gelelektrophorese (Fig. 3). Die Konzentrationen von mRNAs wurden mittels BioPhotometer6131 (Eppendorf) gemessen.

### 4. In vitro Translation von in vitro transkribierten mRNAs

Die Qualität der *in vitro* transkribierten mRNA wurde indirekt durch *in vitro* Translation mit dem Retic Lysate Kit (Ambion) nach Lumineszenzmessung für die Luciferase oder Western Blot Analyse für Elastin bestimmt. Die *in vitro* Translationsreaktionen wurden gemäß den Anweisungen des Herstellers angesetzt.

### 5. Zelllinien und Chemikalien für die Transfektion von IVT-mRNAs

Für die ersten Versuche wurden die Lungenkarzinomzelllinien A549 und SK-MES verwendet. Die Endothelzelle/A549-Hydridomzelllinie EA-hy926 wurde verwendet, um effektive Transfektionsmethoden für Endothelzellen und Gewebe zu etablieren.

Sämtliche mRNA-Transfektionen erfolgten unter Verwendung des Transfektionsreagenz Lipofectamine2000 (Invitrogen/Life Technologies). Als Medium für die Transfektionen wurde OptiMEM (Gibco/Life Technologies) verwendet. Die Negativkontrollen für sämtliche Experimente waren OptiMEM mit gleichen Mengen von Lipofectamine2000, die für die mRNA-Transfektionen verwendet wurden, oder mRNA ohne Transfektionsreagenz in OptiMEM.

### 6. Tranfektion von IVT-mRNAs in Zellkultur

Für die Transfektionen mit Elastin-mRNA wurden die Zellen mit einer Dichte von 500.000 Zellen pro Vertiefung in Platten mit 6 Vertiefungen einen Tag vor den Experimenten ausplattiert.

Die Transfektion erfolgte mit 5 oder 10 µg Elastin-mRNA und 3,3 µL Transfektionsreagenz pro Vertiefung verdünnt in OptiMEM, basierend auf den Angaben des Herstellers.

Das Medium mit den mRNA-Transfektionskomplexen wurde zu den Zellen hinzugegeben und die Platten wurden für 4 Stunden under Zellkultivierungsbedingungen inkubiert. Anschließend wurden 2/3 der Transfektionsgemische durch frisches Zellkulturmedium ersetzt und die Zellen wurden über Nacht inkubiert. Dieses Transfektionsverfahren wurde für die folgenden 2 Tage wiederholt. Am dritten Tag wurden die Transfektionskomplexe vollständig durch frisches Zellkulturmedium ersetzt.

Medium und Zellen wurden einen Tag nach der letzten Transfektion analysiert.

### 7. Expression des Luciferase-Reportergens in vitro

Die erste Bestimmung der Luciferase-Expression erfolgte 5 Stunden nach der Transfektion und anschließend nach jedem Tag, bis die Expression abnahm. Zur Messung der Lumineszenz, die die Luciferase-Expression repräsentiert, wurden 20 µL Medium aus jeder Vertiefung 6/24/48/72 Stunden und 5/10/25 Tage nach Transfektion entnommen, wobei das Medium nach jeder Probenentnahme ausgetauscht wurde.

### 8. Messung der Luciferase-Aktivität

Die Aktivität des Luciferaseenzyms wurde direkt nach der *in vitro* Translation oder 5 Stunden bis 30 Tagen nach der Transfektion von IVT-mRNA in die Zellen gemessen, indem 100 µL von 2,5 ng/µL Substrat Coelenterazin zu 20 µL Zellmedium aus den transfizierten Zellen bzw. zu der *in vitro* Translationsreaktion hinzugegeben wurden. Die resultierende Lumineszenz der Proben wurde in einem Mikroplatten-Lesegerät (Mithras LB 940, Berthold Technologies) gemessen. Die Fig. 4 zeigt die gemessene Lumineszenz in dem Medium der IVT-mRNA transfizierten EA.hy926-Zellen, die die Expression der Luciferase widerspiegelt.

Die Ergebnisse zeiegen, dass schon eine geringen menge von 200 ng IVT-Luciferase-mRNA bereits nach kurzer Inkubationszeit von 5 Stunden eine deutliche Luciferaseexpression induzieren kann. Eine höhere Expression kann mit 1 µg IVT-mRNA bis zu 24 Stunden nach Transfektion erzielt werden, jedoch unterscheidet sich der anschließende Expressionsverlauf nicht mehr von den Proben mit geringerer Menge an IVT-mRNA. Auch eine weitere Erhöhung der Menge transfizierter IVT-mRNA auf 2 µg führt nicht zu einer erhöhten Expression.

### 9. Detektion von (Tropo-)Elastin durch Western Blot

Die Proteine aus den *in vitro* Translationsreaktionen wurden auf 8%iger SDS-PAGE aufgetrennt und zur Immundetektion auf eine Nitrozellulosemembran geblottet. Der primäre Antikörper war ein polyklonaler ELN-Antikörper aus dem Kaninchen (zentraler Bereich) von Abgent, und der Sekundärantikörper war ein Anti-Kaninchen IgG (gesamtes Molekül) aus der Ziege, konjugiert mit alkalischer Phosphatase, von Sigma-Aldrich. Das Elastin-Protein wurde sichtbar gemacht durch Präzipitation des Indigofarbstoffes resultierend aus der NBT/BCIP-Reaktion mit alkalischer Phosphatase (Fig. 5).

Der Nachweis von Elastin nach *in vitro* Translation, die auf Grundlage der IVT-Elastin-mRNA erfolgte, bestätigt die Integrität der erfindungsgemäßen mRNA. Es wird davon ausgegangen, dass ein Proteinnachweis nur erfolgen kann, wenn das synthetisierte Protein den Strukturen entspricht, gegen die der Antikörper entwickelt worden ist. Daher muss die erfindungsgemäße mRNA auch vollständig und intakt zur Proteinsynthese vorgelegen haben. Zwar gibt es offenbar einen unspezifischen Nachweis von Proteinen, die im in-vitro-Translationsmix vorliegen, jedoch ist die Spezifität der Elastin-Bande eindeutig, da sie nicht in der Negativkontrolle, die nur den in-vitro-Translationsmix ohne IVT-mRNA enthält, vorliegt.

### 10. Detektion der Expression von Tropoelastin in der Zellkultur

Die Expression von Elastin wurde mit dem Fastin™ Elastin Assay (Biocolor life science assays) gemäß den Angaben des Herstellers analysiert. Die Fig. 6 zeigt die Menge von Tropoelastin, die 24 Stunden nach der letzten Transfektion mit Elastin-IVT-mRNAs, die verschiedene Anteile von modifizierten Nucleinsäuren enthielten, isoliert wurden.

Nach 3-facher Transfektion von IVT-Elastin-mRNA konnte in den Zellen eine deutliche Expression von Tropoelastin, d.h. von löslichem nicht quervernetzte Elastin, nachgewiesen werden. Es wird deutlich, dass eine besonders große Menge von 10 µg transfizierter IVT-ELN-mRNA keinen erhöhenden Effekt auf die Elastinexpression gegenüber nur 5 µg hat. Auch der höhere Anteil an modifizierten Nukleotiden hat keinen positiven Einfluss auf die Expression. Somit scheint es, dass 5 µg der IVT-ELN-mRNA mit 25 % modifizierten CTP/UTP eine ausreichend, nachweisbare Expression von Elastin bewirken kann.

In einem weiteren Experiment wurden 3 x 10⁵ Zellen pro Vertiefung einer 6-Vertiefungen-Platte eingesät. Am nächsten Tag wurden die Überstände abgesaugt und die Zellen mit 1 ml PBS gewaschen. Anschließend erfolgte eine 4 stündige Inkubation mit Opti-MEM (M), Opti-MEM mit Lipofectamine 2000 (M+L), und Opti-MEM mit Lipofectamine 2000 und 2,5 µg Elastin-mRNA (100% 5mCTP/ΨUTP). Zellüberstände wurden nach 24 und 48 Stunden gesammelt. Die Überstände wurde mittels Dot-Blot analysiert. Das Ergebnis ist in der Fig. 7 dargestellt. Mittels Elastin-spezifischer AK erfolgte der Nachweis von Elastin. Die Zellen mit Elastin-mRNA-Inkubation (M+L+Elastin mRNA) zeigten eine deutlich stärkere Färbung als die Zellen ohne mRNA-Transfektion (M, M+L). Die EA.hy926-Zellen synthetisieren ohne Elastin-mRNA geringe Mengen an Elastin, jedoch durch die Elastin-mRNA-Transfektion wird die Elastin Synthese deutlich verstärkt.

### 11. Beurteilung der Immunogenität der IVT-mRNAs

Transfektionen zur Analyse von Cytokinen und anderen Markern der Immunaktivierung erfolgten entsprechend den oben beschriebenen mRNA-Transfektionen. Zusätzlich wurde das Immunstimulans Polyiosin/Polycytidylsäure (Sigma-Aldrich) bei 100 ng/Vertiefung als Positivkontrolle für die Cytokinaktivierung transfiziert. Die Zellen wurden mit dem Transfektionsmix unter Zellkulturbedingungen inkubiert und das Medium wurde nach 3 Stunden ersetzt.

Am folgenden Tag wurden die Zellen lysiert und die RNA wurde mit Aurum Total RNA Mini Kit (Biorad) extrahiert. Die RNA-Konzentration wurde gemessen und von jeder Probe wurden 40 ng für die cDNA-Synthese mit dem iScript cDNA-Synthesekit (Biorad) verwendet. Die generierte cDNA wurde verdünnt und in quantitative Echtzeit(qRT)-PCR-Reaktionen mit dem iQ SYBR Green Supermix (Biorad) in Dreifachansätzen für jede Probe eingesetzt, kombiniert mit einem spezifischen Primerpaar für IFNa, IFN-γ, IL-1B, IL-12, IL-6, IL-8, TNF-α und einem GAPDH-spezifischen Primerpaar. Zur Quantifizierung der Spiegel der Immunmarkerexpression erfolgte in dem CFX Connect Real-Time PCR Detektionskit (Biorad) eine qRT-PCR in Platten mit 96 Vertiefungen (Fig. 8). Die Ergebnisse zeigen, dass die in-vitro synthetisierte Elastin-mRNA in diesem hochsensitiven Assay nur eine sehr geringe Aktivierung von Cytokinen bewirkt.

### 12. Beschichtung von Koronarstents mit eGFP-mRNA

In einem weiteren Experiment wurde die *in vivo* Expression von eGFP über IVT-mRNA, mit der Koronarstents beschichtet waren, untersucht. Die in vitro Synthese der eGFP-mRNA erfolgte mit dem Plasmidkonstrukt pcDNA3.3_eGFP, wie in *Warren et al.* (a.a.O.) beschrieben. Das Plasmid wurde von den Autoren über Addgene (Cambridge, MA, USA) bereitgestellt.

BMS-Koronarstents 3 x 20 mm von Qualimed (Winsen, Deutschland) wurden dip-beschichtet, und zwar in einer Emulsion mit 70 µg IVT-eGFP-mRNA, komplexiert mit 20 µL Lipofectamin2000, in Nuclease-freiem Wasser und 150 µg Polylactid-co-Glycolid RESOMER® RG 502 H (Sigma Aldrich), gelöst in Ethylacetat.

Die Studie erfolgte in Übereinstimmung mit dem deutschen Tierschutzgesetz und den Empfehlungen über die Haltung und die Verwendung von Labortieren postuliert von der FELASA (Federation of European Laboratoy Animal Science Associations). Sämtliche Protokolle und Verfahren wurden von der Tierschutzkommission der Universität Tübingen genehmigt.

Für diese Studie wurden zwei weibliche Schweine von etwa 65 kg (deutsche Landrasse) aus einer lokalen "spezifisch pathogenfreien" (SPF) Zuchtanstalt verwendet und in die Analyse einbezogen. Nach Ankunft in den Tiereinrichtungen der Universität Tübingen konnten die Tiere eine Woche adaptieren, bevor es zur Intervention kam. Während dieser Zeit wurden klinische Studien durchgeführt, um den Gesundheitsstatus sicherzustellen, insbesondere in Bezug auf das Herz-Kreislauf-System.

Die Stents wurden über einen Ballonkatheter (3 mm) in die linke und rechte Koronararterie jedes Schweins implantiert und expandiert. Die Lokalisierung der Stents wurde mit Hilfe eines Röntgenapparats (C-Bogen) und röntgendichtem Material dargestellt. Eine Überdehnung der Arterien wurde absichtlich vorgenommen. Nach der Implantation erhielten die Tiere Heparin und Clopidogrel, um postoperative Thrombosen zu vermeiden.

44 Stunden nach der Implantation der beschichteten Stents wurden die Schweine eingeschläfert. Die "gestenteten" Gefäße wurden isoliert und über Nacht in 4 % Formaldehyd fixiert.

Für die Fluoreszenzanalyse wurden die Stents in Methylmetacrylat basierendem Einbettungssystem Technovit® 9100 von HeraeusKulzer (Wehrheim, Deutschland) eingebettet, und über FIDSAM (fluorescence intensity decay shape analysis microscopy) beim Institut für Analytische Chemie der Universität Tübingen analysiert.

Das Ergebnis ist in Fig. 9 gezeigt. Dort ist der Teil des Fluoreszenzgewebes dargestellt, der nach der Subtraktion der Autofluoreszenz sichtbar ist. Diese Fluoreszenz basiert ausschließlich auf der induzierten eGFP-Expression und ist deshalb ein Beleg für die effiziente Aufnahme und Translation der IVT-mRNA, die für eGFP codiert, durch die Zellen, die das Stentmaterial umgeben.

### 13. Schweine-Haut-Modell

Es wurde ein Schweine-Haut-Modell etabliert, um die Synthese des mRNA-induzierten Elastins in der Haut nachzuweisen. In den ersten Versuchen wurden 2,5 µg Luciferase mRNA/Lipofectamin 2000 Komplexe in die Haut injiziert. Die Haut wurde nach 24 h zerkleinert und zur Isolierung von Luciferase wurden die Zellen lysiert. Das Ergebnis ist in Fig. 10 gezeigt. Mittels Luciferase Assay wurde die erfolgreiche Transfektion der Zellen mit Luciferase mRNA nachgewiesen.

**Sequenzen**

| | |
|---|---|
| SEQ ID Nr. 1: | Nucleotidsequenz der IVT-Elastin-mRNA (wie in den Ausführungsbeispielen verwendet) |
| SEQ ID Nr. 2: | Nucleotidsequenz der cDNA, abgeleitet von der mRNA des humanen Elastins, Transkriptvariante 1 (NM_000501.2) |
| SEQ ID Nr. 3: | Nucleotidsequenz der cDNA, abgeleitet von der mRNA des humanen Elastins, Transkriptvariante 2 (NM_001081752.1) |
| SEQ ID Nr. 4: | Nucleotidsequenz der cDNA, abgeleitet von der mRNA des humanen Elastins, Transkriptvariante 3 (NM_001081753.1) |
| SEQ ID Nr. 5: | Nucleotidsequenz der cDNA, abgeleitet von der mRNA des humanen Elastins, Transkriptvariante 4 (NM_001081754.1) |
| SEQ ID Nr. 6: | Nucleotidsequenz der cDNA, abgeleitet von der mRNA des humanen Elastins, Transkriptvariante 5 (NM_001081755.1) |
| SEQ ID Nr. 7: | Aminosäuresequenz des Elastins Isoform a [Homo sapiens] (NP_00049.2) |
| SEQ ID Nr. 8: | Aminosäuresequenz des Elastins Isoform b [Homo sapiens] (NP_001075221.1) |
| SEQ ID Nr. 9: | Aminosäuresequenz des Elastins Isoform c [Homo sapiens] (NP_001075222.1) |
| SEQ ID Nr. 10: | Aminosäuresequenz des Elastins Isoform d [Homo sapiens] (NP_001075223.1) |
| SEQ ID Nr. 11: | Aminosäuresequenz des Elastins Isoform e [Homo sapiens] (NP_001075224.1) |

## Patentansprüche

1. Polyribonucleotid codierend für Elastin/Tropoelastin mit einer Nucleotidsequenz, die zumindest eine das Polyribonucleotid stabilisierende chemische Modifizierung aufweist, wobei zumindest ca. 25 % der Uridine des Polyribonucleotids durch Pseudouridine ersetzt sind, und zumindest ca. 25 % der Cytidine des Polyribonucleotids durch 5-Methylcytidine ersetzt sind, um das Polyribonucleotid zu stabilisieren.

2. Polyribonucleotid nach Anspruch 1, **dadurch gekennzeichnet, dass** es eine mRNA ist.

3. Polyribonucleotid nach einem der vorherigen Ansprüche, das ferner eine chemische Modifizierung aufweist, die ausgewählt ist aus der Gruppe bestehend aus: 5'-Cap-Struktur, vorzugsweise eine 5'-Guanin-Kappe, Poly-(A)-Schwanz, ein Kappenstrukturanalog [anti-reverse cap analog (ARCA; 3'O-Me-m⁷G(5')ppp(5')ppp(5')G)], eine Verstärkung der Translations-Initiations-Sequenz beim Startcodon AUG, vorzugsweise durch die Sequenz (CCCCGC)aucGagAUG.

4. Polyribonucleotid nach einem der vorherigen Ansprüche, das die Sequenz SEQ ID Nr. 1 aufweist, bei der zumindest ca. 25 % der Uridine durch Pseudouridine ersetzt sind, und bei der zumindest ca. 25 % der Cytidine durch 5-Methylcytidine ersetzt sind.

5. Polyribonucleotid nach einem der vorherigen Ansprüche, das für eine Aminosäuresequenz codiert, die ausgewählt ist aus der Gruppe bestehend aus: SEQ ID Nr. 7, SEQ ID Nr. 8, SEQ ID Nr. 9, SEQ ID Nr. 10, und SEQ ID Nr. 11.

6. Polyribonucleotid nach einem der vorherigen Ansprüche zur Induktion der Synthese von Elastin/Tropoelastin zur Verwendung bei der Behandlung einer defizitären Synthese von elastischem Faserprotein, insbesondere bei altersbedingtem Elastizitätsverlust der Haut (Faltenbildung), bei der Förderung der Wundheilung, bei der Wiedererlangung der Elastizität, vorzugsweise von Vagilagewebe, Weich- und Hartgewebe des Priodontiums.

7. Polyribonucleotid nach einem der vorherigen Ansprüche zur Verwendung bei der Behandlung einer Krankheit, die ausgewählt ist aus der Gruppe bestehend aus: Arteriosklerose, Aortenstenose, Aortenaneurysma, Lungenemphysem, Dermatochalasis, Williams-Beuren-Syndrom, subvalvuläre angeborene Aortenstenose (SVAS).

8. Kosmetische Zusammensetzung, die das Polyribonucleotid nach einem der Ansprüche 1 bis 7 und eine kosmetisch akzeptable Formulierung und ggf. weitere Wirk- und Hilfsstoffe aufweist.

9. Pharmazeutische Zusammensetzung, die das Polyribonucleotid nach einem der Ansprüche 1 bis 7 und eine pharmazeutisch akzeptable Formulierung und ggf. weitere Wirk- und Hilfsstoffe aufweist.

10. Zusammensetzung nach Anspruch 8 oder 9, **dadurch gekennzeichnet, dass** sie zusätzlich ein Immunsuppressivum, vorzugsweise einen Interferon-Inhibitor aufweist.

11. Medizinprodukt, vorzugsweise medizinisches Patch oder Implantat, weiter vorzugsweise Stent/Gefäßimplantat, das das Polyribonucleotid nach einem der Ansprüche 1 bis 7 und/oder die Zusammensetzung nach einem der Ansprüche 8 bis 10 aufweist, vorzugsweise damit beschichtet ist.

## Claims

1. Polyribonucleotide encoding elastin/tropoelastin having a nucleotide sequence comprising at least one said polyribonucleotide stabilizing chemical modification, wherein at least approx. 25 % of the uridines of said polyribonucleotide are replaced by pseudouridines, and at least approx. 25 % of the cytidines of said polynucleotide are replaced by 5-methylcytidine, to stabilize said polyribonucleotide.

2. Polyribonucleotide of claim 1, **characterized in that** it is an mRNA.

3. Polyribonucleotide of any of the preceding claims, further comprising a chemical modification is selected from the group consisting of: 5' cap structure, preferably a 5' guanine cap, poly (A) tail, a cap structure analog [anti-reverse cap analog (ARCA; 3'0-Me-m⁷G(5')ppp(5')ppp(5')G)], a strengthening of the translation-initiation sequence at the start codon AUG, e.g. by the sequence (CCCCGC)aucGagAUG.

4. Polyribonucleotide of any of the preceding claims, comprising the sequence of SEQ ID No. 1, where at least approx. 25 % of the uridines are replaced by pseudouridines, and/or where at least approx. 25 % of the cytidines are replaced by 5-methylcytidine.

5. Polyribonucleotide of any of the preceding claims, encoding an amino acid sequence selected from the group consisting of: SEQ ID No. 7, SEQ ID No. 8, SEQ ID No. 9, SEQ ID No. 10, and SEQ ID No. 11.

6. Polyribonucleotide of any of the preceding claims, for the induction of the synthesis of elastin/pseudoelastin for the treatment of a deficient synthesis of elastic fibrous protein in particular in age-related loss of elasticity of the skin (wrinkle formation), for promoting the wound healing, for the recovery of the elasticity, preferably of vaginal tissue, soft and hard tissue of the periodontium.

7. Polyribonucleotide of any of the preceding claims for the treatment of a disease selected from the group consisting of: arteriosclerosis, aortic stenosis, aortic aneurysm, pulmonary emphysema, dermatochalasis, Williams-Beuren syndrome, sub valvular innate aortic stenosis (SVAS).

8. Cosmetic composition, comprising the polyribonucleotide of any of the claims 1 to 7 and a cosmetically acceptable formulation and, if applicable, further active agents and excipients.

9. Pharmaceutical composition, comprising the polyribonucleotide of any of the claims 1 to 7 and a pharmaceutically acceptable formulation and, if applicable, additional active agents and excipients.

10. Composition of claim 8 or 9, **characterized in that** it additionally comprises an immuno suppressive agent, preferably an interferon inhibitor.

11. Medical product, preferably a medical patch or implant, further preferably a stent/vascular implant, comprising the polyribonucleotide of any of the claims 1 to 7 and/or the composition of any of the claims 8 to 10, preferably coated therewith.

## Revendications

1. Polyribonucléotide codant pour l'élastine/la tropoélastine avec une séquence de nucléotides qui présente au moins une modification chimique stabilisant le polyribonucléotide, dans lequel au moins environ 25 % des uridines du polyribonucléotide sont remplacées par des pseudouridines et au moins environ 25 % des cytidines du polyribonucléotide sont remplacées par des 5-méthylcytidines pour stabiliser le polyribonucléotide.

2. Polyribonucléotide selon la revendication 1, **caractérisé en ce qu'**il est un ARNm

3. Polyribonucléotide selon l'une quelconque des revendications précédentes, qui présente en outre une modification chimique, qui est choisie parmi le groupe constitué de : structure de coiffe en 5', de préférence d'une coiffe de guanine en 5', queue poly(A), d'un analogue de structure de coiffe (analogue de coiffe anti-retour (ARCA ; 3'O-Me-m⁷G(5')ppp(5')ppp(5')G)], d'une amplification de la séquence d'initiation de traduction pour le codon d'initiation AUG, de préférence par la séquence (CCCCGC)aucGagAUG.

4. Polyribonucléotide selon l'une quelconque des revendications précédentes, qui présente la séquence SEQ ID n°1, où les au moins environ 25 % des uridines sont remplacées par des pseudouridines et où les au moins 25 % des cytidines sont remplacées par des 5-méthylcytidines.

5. Polyribonucléotide selon l'une quelconque des revendications précédentes, qui code pour une séquence d'acides aminés, qui est choisie parmi le groupe constitué de : SEQ ID n°7, SEQ ID n°8, SEQ ID n°9, SEQ ID n°10 et SEQ ID n°11.

6. Polyribonucléotide selon l'une quelconque des revendications précédentes servant à l'induction de la synthèse d'élastine/de tropoélastine aux fins de l'utilisation lors du traitement d'une insuffisance de synthèse de protéine de fibres élastique, en particulier lors de la perte d'élasticité liée au vieillissement de la peau (formation de rides), lors de la stimulation de la cicatrisation, lors du rétablissement de l'élasticité, de préférence de tissus vaginaux, de tissus mous et de tissus durs du parodonte.

7. Polyribonucléotide selon l'une quelconque des revendications précédentes destiné à être utilisé lors du traitement d'une maladie, qui est choisie parmi le groupe constitué de : artériosclérose, sténose aortique, anévrisme aortique, emphysème pulmonaire, dermatochalasis, syndrome de Williams-Beuren, sténose aortique congénitale sous-valvulaire (SVAS).

8. Composition cosmétique, qui présente le polyribonucléotide selon l'une quelconque des revendications 1 à 7 et une formulation cosmétiquement acceptable et éventuellement d'autres principes actifs et adjuvants.

9. Composition pharmaceutique, qui présente le polyribonucléotide selon l'une quelconque des revendications 1 à 7 et une formulation cosmétiquement acceptable et éventuellement d'autres principes actifs et adjuvants.

10. Composition selon la revendication 8 ou 9, **caractérisée en ce qu'**elle présente en supplément un immunosuppresseur, de préférence un inhibiteur d'interféron.

11. Produit médical, de préférence patch ou implant médical, de manière davantage préférée endoprothèse/implant vasculaire, qui présente le polyribonucléotide selon l'une quelconque des revendications 1 à 7 et/ou la composition selon l'une quelconque des revendications 8 à 10, de préférence qui en est revêtu.


### SEQUENZPROTOKOLL

<110> Eberhard-Karls-Universität Tübingen, Medizinische Fakultät
<120> Polyribonucleotid
<130> 5402P503WO
<160> 11
<170> PatentIn Version 3.5
<210> 1
   <211> 3196
   <212> RNA
   <213> Künstliche Sequenz
<220>
   <223> Sonstige Angaben
<400> 1
<210> 2
   <211> 3480
   <212> DNA
   <213> Künstliche Sequenz
<220>
   <223> Sonstige Angaben
<400> 2
<210> 3
   <211> 3339
   <212> DNA
   <213> Künstliche Sequenz
<220>
   <223> Sonstige Angaben
<400> 3
   ctccctcttt ccctcacagc cgacgaggca acaattaggc tttggggata aaacgaggtg      60
   cggagagcgg gctggggcat ttctccccga gatggcgggt ctgacggcgg cggccccgcg     120
   gcccggagtc ctcctgctcc tgctgtccat cctccacccc tctcggcctg gaggggtccc     180
   tggggccatt cctggtggag ttcctggagg agtcttttat ccagcgctgg ggcctggagg     240
   caaacctctt aagccagttc ccggagggct tgcgggtgct ggccttgggg cagggctcgg     300
   cgccttcccc gcagttacct ttccgggggc tctggtgcct ggtggagtgg ctgacgctgc     360
   tgcagcctat aaagctgcta aggctggcgc tgggcttggt ggtgtcccag gagttggtgg     420
   cttaggagtg tctgcaggtg cggtggttcc tcagcctgga gccggagtga agcctgggaa     480
   agtgccgggt gtggggctgc caggtgtata cccaggtggc gtgctcccag gagctcggtt     540
   ccccggtgtg ggggtgctcc ctggagttcc cactggagca ggagttaagc ccaaggctcc      600
   aggtgtaggt ggagcttttg ctggaatccc aggagttgga ccctttgggg gaccgcaacc     660
   tggagtccca ctggggtatc ccatcaaggc ccccaagctg cctggtggct atggactgcc     720
   ctacaccaca gggaaactgc cctatggcta tgggcccgga ggagtggctg gtgcagcggg     780
   caaggctggt tacccaacag ggacaggggt tggcccccag gcagcagcag cagcggcagc     840
   taaagcagca gcaaagttcg gtgctggagc agccggagtc ctccctggtg ttggaggggc     900
   tggtgttcct ggcgtgcctg gggcaattcc tggaattgga ggcatcgcag gcgttgggac     960
   tccagctgca gctgcagctg cagcagcagc cgctaaggca gccaagtatg gagctgctgc    1020
   aggcttagtg cctggtgggc caggctttgg cccgggagta gttggtgtcc caggagctgg    1080
   cgttccaggt gttggtgtcc caggagctgg gattccagtt gtcccaggtg ctgggatccc    1140
   aggtgctgcg gttccagggg ttgtgtcacc agaagcagct gctaaggcag ctgcaaaggc    1200
   agccaaatac ggggccaggc ccggagtcgg agttggaggc attcctactt acggggttgg    1260
   agctgggggc tttcccggct ttggtgtcgg agtcggaggt atccctggag tcgcaggtgt    1320
   ccctggtgtc ggaggtgttc ccggagtcgg aggtgtcccg ggagttggca tttcccccga    1380
   agctcaggca gcagctgccg ccaaggctgc caagtacggg ttagttcctg gtgtcggcgt    1440
   ggctcctgga gttggcgtgg ctcctggtgt cggtgtggct cctggagttg gcttggctcc    1500
   tggagttggc gtggctcctg gagttggtgt ggctcctggc gttggcgtgg ctcccggcat    1560
   tggccctggt ggagttgcag ctgcagcaaa atccgctgcc aaggtggctg ccaaagccca    1620
   gctccgagct gcagctgggc ttggtgctgg catccctgga cttggagttg gtgtcggcgt 1680
   ccctggactt ggagttggtg ctggtgttcc tggacttgga gttggtgctg gtgttcctgg 1740
   cttcggggca gtacctggag ccctggctgc cgctaaagca gccaaatatg gagcagcagt 1800
   gcctggggtc cttggagggc tcggggctct cggtggagta ggcatcccag gcggtgtggt 1860
   gggagccgga cccgccgccg ccgctgccgc agccaaagct gctgccaaag ccgcccagtt 1920
   tggcctagtg ggagccgctg ggctcggagg actcggagtc ggagggcttg gagttccagg 1980
   tgttgggggc cttggaggta tacctccagc tgcagccgct aaagcagcta aatacggagt 2040
   ggcagcaaga cctggcttcg gattgtctcc cattttccca ggtggggcct gcctggggaa 2100
   agcttgtggc cggaagagaa aatgagcttc ctaggacccc tgactcacga cctcatcaac 2160
   gttggtgcta ctgcttggtg gagaatgtaa accctttgta accccatccc atgcccctcc 2220
   gactccccac cccaggaggg aacgggcagg ccgggcggcc ttgcagatcc acagggcaag 2280
   gaaacaagag gggagcggcc aagtgccccg accaggaggc cccctacttc agaggcaagg 2340
   gccatgtggt cctggccccc caccccatcc cttcccacct aggagctccc cctccacaca 2400
   gcctccatct ccaggggaac ttggtgctac acgctggtgc tcttatcttc ctggggggag 2460
   ggaggaggga agggtggccc ctcggggaac cccctacctg gggctcctct aaagatggtg 2520
   cagacacttc ctgggcagtc ccagctcccc ctgcccacca ggacccaccg ttggctgcca 2580
   tccagttggt acccaagcac ctgaagcctc aaagctggat tcgctctagc atccctcctc 2640
   tcctgggtcc acttggccgt ctcctcccca ccgatcgctg ttccccacat ctggggcgct 2700
   tttgggttgg aaaaccaccc cacactggga atagccacct tgcccttgta gaatccatcc 2760
   gcccatccgt ccattcatcc atcggtccgt ccatccatgt ccccagttga ccgcccggca 2820
   ccactagctg gctgggtgca cccaccatca acctggttga cctgtcatgg ccgcctgtgc 2880
   cctgcctcca cccccatcct acactccccc agggcgtgcg gggctgtgca gactggggtg 2940
   ccaggcatct cctccccacc cggggtgtcc ccacatgcag tactgtatac cccccatccc 3000
   tccctcggtc cactgaactt cagagcagtt cccattcctg ccccgcccat ctttttgtgt 3060
   ctcgctgtga tagatcaata aatattttat tttttgtcct ggatatttgg ggattatttt 3120
   tgattgttga tattctcttt tggttttatt gttgtggttc attgaaaaaa aaagataatt 3180
   tttttttctg atccggggag ctgtatcccc agtagaaaaa acattttaat cactctaata 3240
   taactctgga tgaaacacac cttttttttt aataagaaaa gagaattaac tgcttcagaa 3300
   atgactaata aatgaaaaac ctttaaagga aaaaaaaaa 3339
<210> 4
   <211> 3384
   <212> DNA
   <213> Künstliche Sequenz
<220>
   <223> Sonstige Angaben
<400> 4
   ctccctcttt ccctcacagc cgacgaggca acaattaggc tttggggata aaacgaggtg 60
   cggagagcgg gctggggcat ttctccccga gatggcgggt ctgacggcgg cggccccgcg 120
   gcccggagtc ctcctgctcc tgctgtccat cctccacccc tctcggcctg gaggggtccc 180
   tggggccatt cctggtggag ttcctggagg agtcttttat ccaggggctg gtctcggagc 240
   ccttggagga ggagcgctgg ggcctggagg caaacctctt aagccagttc ccggagggct 300
   tgcgggtgct ggccttgggg cagggctcgg cgccttcccc gcagttacct ttccgggggc 360
   tctggtgcct ggtggagtgg ctgacgctgc tgcagcctat aaagctgcta aggctggcgc 420
   tgggcttggt ggtgtcccag gagttggtgg cttaggagtg tctgcagccc cttctgtgcc 480
   aggtgcggtg gttcctcagc ctggagccgg agtgaagcct gggaaagtgc cgggtgtggg 540
   gctgccaggt gtatacccag gtggcgtgct cccaggagct cggttccccg gtgtgggggt 600
   gctccctgga gttcccactg gagcaggagt taagcccaag gctccaggtg taggtggagc 660
   ttttgctgga atcccaggag ttggaccctt tgggggaccg caacctggag tcccactggg 720
   gtatcccatc aaggccccca agctgcctgg tggctatgga ctgccctaca ccacagggaa 780
   actgccctat ggctatgggc ccggaggagt ggctggtgca gcgggcaagg ctggttaccc 840
   aacagggaca ggggttggcc cccaggcagc agcagcagcg gcagctaaag cagcagcaaa 900
   gttcggtgct ggagcagccg gagtcctccc tggtgttgga ggggctggtg ttcctggcgt 960
   gcctggggca attcctggaa ttggaggcat cgcaggcgtt gggactccag ctgcagctgc 1020
   agctgcagca gcagccgcta aggcagccaa gtatggagct gctgcaggct tagtgcctgg 1080
   tgggccaggc tttggcccgg gagtagttgg tgtcccagga gctggcgttc caggtgttgg 1140
   tgtcccagga gctgggattc cagttgtccc aggtgctggg atcccaggtg ctgcggttcc 1200
   aggggttgtg tcaccagaag cagctgctaa ggcagctgca aaggcagcca aatacggggc 1260
   caggcccgga gtcggagttg gaggcattcc tacttacggg gttggagctg gggctttccc 1320
   cggctttggt gtcggagtcg gaggtatccc tggagtcgca ggtgtccctg gtgtcggagg 1380
   tgttcccgga gtcggaggtg tcccgggagt tggcatttcc cccgaagctc aggcagcagc 1440
   tgccgccaag gctgccaagt acgggttagt tcctggtgtc ggcgtggctc ctggagttgg 1500
   cgtggctcct ggtgtcggtg tggctcctgg agttggcttg gctcctggag ttggcgtggc 1560
   tcctggagtt ggtgtggctc ctggcgttgg cgtggctccc ggcattggcc ctggtggagt 1620
   tgcagctgca gcaaaatccg ctgccaaggt ggctgccaaa gcccagctcc gagctgcagc 1680
   tgggcttggt gctggcatcc ctggacttgg agttggtgtc ggcgtccctg gacttggagt 1740
   tggtgctggt gttcctggac ttggagttgg tgctggtgtt cctggcttcg gggcagtacc 1800
   tggagccctg gctgccgcta aagcagccaa atatggagca gcagtgcctg gggtccttgg 1860
   agggctcggg gctctcggtg gagtaggcat cccaggcggt gtggtgggag ccggacccgc 1920
   cgccgccgct gccgcagcca aagctgctgc caaagccgcc cagtttggcc tagtgggagc 1980
   cgctgggctc ggaggactcg gagtcggagg gcttggagtt ccaggtgttg ggggccttgg 2040
   aggtatacct ccagctgcag ccgctaaagc agctaaatac ggagtggcag caagacctgg 2100
   cttcggattg tctcccattt tcccaggtgg ggcctgcctg gggaaagctt gtggccggaa 2160
   gagaaaatga gcttcctagg acccctgact cacgacctca tcaacgttgg tgctactgct 2220
   tggtggagaa tgtaaaccct ttgtaacccc atcccatgcc cctccgactc cccaccccag 2280
   gagggaacgg gcaggccggg cggccttgca gatccacagg gcaaggaaac aagaggggag 2340
   cggccaagtg ccccgaccag gaggccccct acttcagagg caagggccat gtggtcctgg 2400
   ccccccaccc catcccttcc cacctaggag ctccccctcc acacagcctc catctccagg 2460
   ggaacttggt gctacacgct ggtgctctta tcttcctggg gggagggagg agggaagggt 2520
   ggcccctcgg ggaaccccct acctggggct cctctaaaga tggtgcagac acttcctggg 2580
   cagtcccagc tccccctgcc caccaggacc caccgttggc tgccatccag ttggtaccca 2640
   agcacctgaa gcctcaaagc tggattcgct ctagcatccc tcctctcctg ggtccacttg 2700
   gccgtctcct ccccaccgat cgctgttccc cacatctggg gcgcttttgg gttggaaaac 2760
   caccccacac tgggaatagc caccttgccc ttgtagaatc catccgccca tccgtccatt 2820
   catccatcgg tccgtccatc catgtcccca gttgaccgcc cggcaccact agctggctgg 2880
   gtgcacccac catcaacctg gttgacctgt catggccgcc tgtgccctgc ctccaccccc 2940
   atcctacact cccccagggc gtgcggggct gtgcagactg gggtgccagg catctcctcc 3000
   ccacccgggg tgtccccaca tgcagtactg tatacccccc atccctccct cggtccactg 3060
   aacttcagag cagttcccat tcctgccccg cccatctttt tgtgtctcgc tgtgatagat 3120
   caataaatat tttatttttt gtcctggata tttggggatt atttttgatt gttgatattc 3180
   tcttttggtt ttattgttgt ggttcattga aaaaaaaaga taattttttt ttctgatccg 3240
   gggagctgta tccccagtag aaaaaacatt ttaatcactc taatataact ctggatgaaa 3300
   cacacctttt tttttaataa gaaaagagaa ttaactgctt cagaaatgac taataaatga 3360
   aaaaccttta aaggaaaaaa aaaa 3384
<210> 5
   <211> 3441
   <212> DNA
   <213> Künstliche Sequenz
<220>
   <223> Sonstige Angaben
<400> 5
   ctccctcttt ccctcacagc cgacgaggca acaattaggc tttggggata aaacgaggtg 60
   cggagagcgg gctggggcat ttctccccga gatggcgggt ctgacggcgg cggccccgcg 120
   gcccggagtc ctcctgctcc tgctgtccat cctccacccc tctcggcctg gaggggtccc 180
   tggggccatt cctggtggag ttcctggagg agtcttttat ccaggggctg gtctcggagc 240
   ccttggagga ggagcgctgg ggcctggagg caaacctctt aagccagttc ccggagggct 300
   tgcgggtgct ggccttgggg cagggctcgg cgccttcccc gcagttacct ttccgggggc 360
   tctggtgcct ggtggagtgg ctgacgctgc tgcagcctat aaagctgcta aggctggcgc 420
   tgggcttggt ggtgtcccag gagttggtgg cttaggagtg tctgcagccc cttctgtgcc 480
   aggtgcggtg gttcctcagc ctggagccgg agtgaagcct gggaaagtgc cgggtgtggg 540
   gctgccaggt gtatacccag gtggcgtgct cccaggagct cggttccccg gtgtgggggt 600
   gctccctgga gttcccactg gagcaggagt taagcccaag gctccaggtg taggtggagc 660
   ttttgctgga atcccaggag ttggaccctt tgggggaccg caacctggag tcccactggg 720
   gtatcccatc aaggccccca agctgcctgg tggctatgga ctgccctaca ccacagggaa 780
   actgccctat ggctatgggc ccggaggagt ggctggtgca gcgggcaagg ctggttaccc 840
   aacagggaca ggggttggcc cccaggcagc agcagcagcg gcagctaaag cagcagcaaa 900
   gttcggtgct ggagcagccg gagtcctccc tggtgttgga ggggctggtg ttcctggcgt 960
   gcctggggca attcctggaa ttggaggcat cgcaggcgtt gggactccag ctgcagctgc 1020
   agctgcagca gcagccgcta aggcagccaa gtatggagct gctgcaggct tagtgcctgg 1080
   tgggccaggc tttggcccgg gagtagttgg tgtcccagga gctggcgttc caggtgttgg 1140
   tgtcccagga gctgggattc cagttgtccc aggtgctggg atcccaggtg ctgcggttcc 1200
   aggggttgtg tcaccagaag cagctgctaa ggcagctgca aaggcagcca aatacggggc 1260
   caggcccgga gtcggagttg gaggcattcc tacttacggg gttggagctg gggctttccc 1320
   cggctttggt gtcggagtcg gaggtatccc tggagtcgca ggtgtccctg gtgtcggagg 1380
   tgttcccgga gtcggaggtg tcccgggagt tggcatttcc cccgaagctc aggcagcagc 1440
   tgccgccaag gctgccaagt acggagtggg gaccccagca gctgcagctg ctaaagcagc 1500
   cgccaaagcc gcccagtttg ggttagttcc tggtgtcggc gtggctcctg gagttggcgt 1560
   ggctcctggt gtcggtgtgg ctcctggagt tggcttggct cctggagttg gcgtggctcc 1620
   tggagttggt gtggctcctg gcgttggcgt ggctcccggc attggccctg gtggagttgc 1680
   agctgcagca aaatccgctg ccaaggtggc tgccaaagcc cagctccgag ctgcagctgg 1740
   gcttggtgct ggcatccctg gacttggagt tggtgtcggc gtccctggac ttggagttgg 1800
   tgctggtgtt cctggacttg gagttggtgc tggtgttcct ggcttcgggg cagtacctgg 1860
   agccctggct gccgctaaag cagccaaata tggagcagca gtgcctgggg tccttggagg 1920
   gctcggggct ctcggtggag taggcatccc aggcggtgtg gtgggagccg gacccgccgc 1980
   cgccgctgcc gcagccaaag ctgctgccaa agccgcccag tttggcctag tgggagccgc 2040
   tgggctcgga ggactcggag tcggagggct tggagttcca ggtgttgggg gccttggagg 2100
   tatacctcca gctgcagccg ctaaagcagc taaatacgga gtggcagcaa gacctggctt 2160
   cggattgtct cccattttcc caggtggggc ctgcctgggg aaagcttgtg gccggaagag 2220
   aaaatgagct tcctaggacc cctgactcac gacctcatca acgttggtgc tactgcttgg 2280
   tggagaatgt aaaccctttg taaccccatc ccatgcccct ccgactcccc accccaggag 2340
   ggaacgggca ggccgggcgg ccttgcagat ccacagggca aggaaacaag aggggagcgg 2400
   ccaagtgccc cgaccaggag gccccctact tcagaggcaa gggccatgtg gtcctggccc 2460
   cccaccccat cccttcccac ctaggagctc cccctccaca cagcctccat ctccagggga 2520
   acttggtgct acacgctggt gctcttatct tcctgggggg agggaggagg gaagggtggc 2580
   ccctcgggga accccctacc tggggctcct ctaaagatgg tgcagacact tcctgggcag 2640
   tcccagctcc ccctgcccac caggacccac cgttggctgc catccagttg gtacccaagc 2700
   acctgaagcc tcaaagctgg attcgctcta gcatccctcc tctcctgggt ccacttggcc 2760
   gtctcctccc caccgatcgc tgttccccac atctggggcg cttttgggtt ggaaaaccac 2820
   cccacactgg gaatagccac cttgcccttg tagaatccat ccgcccatcc gtccattcat 2880
   ccatcggtcc gtccatccat gtccccagtt gaccgcccgg caccactagc tggctgggtg 2940
   cacccaccat caacctggtt gacctgtcat ggccgcctgt gccctgcctc cacccccatc 3000
   ctacactccc ccagggcgtg cggggctgtg cagactgggg tgccaggcat ctcctcccca 3060
   cccggggtgt ccccacatgc agtactgtat accccccatc cctccctcgg tccactgaac 3120
   ttcagagcag ttcccattcc tgccccgccc atctttttgt gtctcgctgt gatagatcaa 3180
   taaatatttt attttttgtc ctggatattt ggggattatt tttgattgtt gatattctct 3240
   tttggtttta ttgttgtggt tcattgaaaa aaaaagataa tttttttttc tgatccgggg 3300
   agctgtatcc ccagtagaaa aaacatttta atcactctaa tataactctg gatgaaacac 3360
   accttttttt ttaataagaa aagagaatta actgcttcag aaatgactaa taaatgaaaa 3420
   acctttaaag gaaaaaaaaa a 3441
<210> 6
   <211> 3423
   <212> DNA
   <213> Künstliche Sequenz
<220>
   <223> Sonstige Angaben
<400> 6
   ctccctcttt ccctcacagc cgacgaggca acaattaggc tttggggata aaacgaggtg       60
   cggagagcgg gctggggcat ttctccccga gatggcgggt ctgacggcgg cggccccgcg      120
   gcccggagtc ctcctgctcc tgctgtccat cctccacccc tctcggcctg gaggggtccc      180
   tggggccatt cctggtggag ttcctggagg agtcttttat ccaggggctg gtctcggagc      240
   ccttggagga ggagcgctgg ggcctggagg caaacctctt aagccagttc ccggagggct      300
   tgcgggtgct ggccttgggg cagggctcgg cgccttcccc gcagttacct ttccgggggc      360
   tctggtgcct ggtggagtgg ctgacgctgc tgcagcctat aaagctgcta aggctggcgc      420
   tgggcttggt ggtgtcccag gagttggtgg cttaggagtg tctgcaggtg cggtggttcc      480
   tcagcctgga gccggagtga agcctgggaa agtgccgggt gtggggctgc caggtgtata      540
   cccaggtggc gtgctcccag gagctcggtt ccccggtgtg ggggtgctcc ctggagttcc       600
   cactggagca ggagttaagc ccaaggctcc aggtgtaggt ggagcttttg ctggaatccc      660
   aggagttgga ccctttgggg gaccgcaacc tggagtccca ctggggtatc ccatcaaggc      720
   ccccaagctg cctggtggct atggactgcc ctacaccaca gggaaactgc cctatggcta      780
   tgggcccgga ggagtggctg gtgcagcggg caaggctggt tacccaacag ggacaggggt      840
   tggcccccag gcagcagcag cagcggcagc taaagcagca gcaaagttcg gtgctggagc      900
   agccggagtc ctccctggtg ttggaggggc tggtgttcct ggcgtgcctg gggcaattcc      960
   tggaattgga ggcatcgcag gcgttgggac tccagctgca gctgcagctg cagcagcagc     1020
   cgctaaggca gccaagtatg gagctgctgc aggcttagtg cctggtgggc caggctttgg     1080
   cccgggagta gttggtgtcc caggagctgg cgttccaggt gttggtgtcc caggagctgg     1140
   gattccagtt gtcccaggtg ctgggatccc aggtgctgcg gttccagggg ttgtgtcacc     1200
   agaagcagct gctaaggcag ctgcaaaggc agccaaatac ggggccaggc ccggagtcgg     1260
   agttggaggc attcctactt acggggttgg agctgggggc tttcccggct ttggtgtcgg     1320
   agtcggaggt atccctggag tcgcaggtgt ccctggtgtc ggaggtgttc ccggagtcgg     1380
   aggtgtcccg ggagttggca tttcccccga agctcaggca gcagctgccg ccaaggctgc     1440
   caagtacggg ttagttcctg gtgtcggcgt ggctcctgga gttggcgtgg ctcctggtgt     1500
   cggtgtggct cctggagttg gcttggctcc tggagttggc gtggctcctg gagttggtgt     1560
   ggctcctggc gttggcgtgg ctcccggcat tggccctggt ggagttgcag ctgcagcaaa     1620
   atccgctgcc aaggtggctg ccaaagccca gctccgagct gcagctgggc ttggtgctgg     1680
   catccctgga cttggagttg gtgtcggcgt ccctggactt ggagttggtg ctggtgttcc     1740
   tggacttgga gttggtgctg gtgttcctgg cttcggggca gtacctggag ccctggctgc     1800
   cgctaaagca gccaaatatg gagcagcagt gcctggggtc cttggagggc tcggggctct     1860
   cggtggagta ggcatcccag gcggtgtggt gggagccgga cccgccgccg ccgctgccgc 1920
   agccaaagct gctgccaaag ccgcccagtt tggcctagtg ggagccgctg ggctcggagg 1980
   actcggagtc ggagggcttg gagttccagg tgttgggggc cttggaggta tacctccagc 2040
   tgcagccgct aaagcagcta aatacggtgc tgctggcctt ggaggtgtcc tagggggtgc 2100
   cgggcagttc ccacttggag gagtggcagc aagacctggc ttcggattgt ctcccatttt 2160
   cccaggtggg gcctgcctgg ggaaagcttg tggccggaag agaaaatgag cttcctagga 2220
   cccctgactc acgacctcat caacgttggt gctactgctt ggtggagaat gtaaaccctt 2280
   tgtaacccca tcccatgccc ctccgactcc ccaccccagg agggaacggg caggccgggc 2340
   ggccttgcag atccacaggg caaggaaaca agaggggagc ggccaagtgc cccgaccagg 2400
   aggcccccta cttcagaggc aagggccatg tggtcctggc cccccacccc atcccttccc 2460
   acctaggagc tccccctcca cacagcctcc atctccaggg gaacttggtg ctacacgctg 2520
   gtgctcttat cttcctgggg ggagggagga gggaagggtg gcccctcggg gaacccccta 2580
   cctggggctc ctctaaagat ggtgcagaca cttcctgggc agtcccagct ccccctgccc 2640
   accaggaccc accgttggct gccatccagt tggtacccaa gcacctgaag cctcaaagct 2700
   ggattcgctc tagcatccct cctctcctgg gtccacttgg ccgtctcctc cccaccgatc 2760
   gctgttcccc acatctgggg cgcttttggg ttggaaaacc accccacact gggaatagcc 2820
   accttgccct tgtagaatcc atccgcccat ccgtccattc atccatcggt ccgtccatcc 2880
   atgtccccag ttgaccgccc ggcaccacta gctggctggg tgcacccacc atcaacctgg 2940
   ttgacctgtc atggccgcct gtgccctgcc tccaccccca tcctacactc ccccagggcg 3000
   tgcggggctg tgcagactgg ggtgccaggc atctcctccc cacccggggt gtccccacat 3060
   gcagtactgt atacccccca tccctccctc ggtccactga acttcagagc agttcccatt 3120
   cctgccccgc ccatcttttt gtgtctcgct gtgatagatc aataaatatt ttattttttg 3180
   tcctggatat ttggggatta tttttgattg ttgatattct cttttggttt tattgttgtg 3240
   gttcattgaa aaaaaaagat aatttttttt tctgatccgg ggagctgtat ccccagtaga 3300
   aaaaacattt taatcactct aatataactc tggatgaaac acaccttttt ttttaataag 3360
   aaaagagaat taactgcttc agaaatgact aataaatgaa aaacctttaa aggaaaaaaa 3420
   aaa 3423
<210> 7
   <211> 724
   <212> PRT
   <213> Homo sapiens
<220>
   <223> Sonstige Angaben
<400> 7
   Met Ala Gly Leu Thr Ala Ala Ala Pro Arg Pro Gly Val Leu Leu Leu
   1               5                   10                  15
   Leu Leu Ser Ile Leu His Pro Ser Arg Pro Gly Gly Val Pro Gly Ala
               20                  25                  30
   Ile Pro Gly Gly Val Pro Gly Gly Val Phe Tyr Pro Gly Ala Gly Leu
           35                  40                  45
   Gly Ala Leu Gly Gly Gly Ala Leu Gly Pro Gly Gly Lys Pro Leu Lys
       50                  55                  60
   Pro Val Pro Gly Gly Leu Ala Gly Ala Gly Leu Gly Ala Gly Leu Gly
   65                  70                  75                  80
   Ala Phe Pro Ala Val Thr Phe Pro Gly Ala Leu Val Pro Gly Gly Val
                   85                  90                  95
   Ala Asp Ala Ala Ala Ala Tyr Lys Ala Ala Lys Ala Gly Ala Gly Leu
               100                 105                 110
   Gly Gly Val Pro Gly Val Gly Gly Leu Gly Val Ser Ala Gly Ala Val
           115                 120                 125
   Val Pro Gln Pro Gly Ala Gly Val Lys Pro Gly Lys Val Pro Gly Val
       130                 135                 140
   Gly Leu Pro Gly Val Tyr Pro Gly Gly Val Leu Pro Gly Ala Arg Phe
   145                 150                 155                 160
   Pro Gly Val Gly Val Leu Pro Gly Val Pro Thr Gly Ala Gly Val Lys
                   165                 170                 175
   Pro Lys Ala Pro Gly Val Gly Gly Ala Phe Ala Gly Ile Pro Gly Val
               180                 185                 190
   Gly Pro Phe Gly Gly Pro Gln Pro Gly Val Pro Leu Gly Tyr Pro Ile
           195                 200                 205
   Lys Ala Pro Lys Leu Pro Gly Gly Tyr Gly Leu Pro Tyr Thr Thr Gly
       210                 215                 220
   Lys Leu Pro Tyr Gly Tyr Gly Pro Gly Gly Val Ala Gly Ala Ala Gly
   225                 230                 235                 240
   Lys Ala Gly Tyr Pro Thr Gly Thr Gly Val Gly Pro Gln Ala Ala Ala
                   245                 250                 255
   Ala Ala Ala Ala Lys Ala Ala Ala Lys Phe Gly Ala Gly Ala Ala Gly
               260                 265                 270
   Val Leu Pro Gly Val Gly Gly Ala Gly Val Pro Gly Val Pro Gly Ala
           275                 280                 285
   Ile Pro Gly Ile Gly Gly Ile Ala Gly Val Gly Thr Pro Ala Ala Ala
       290                 295                 300
   Ala Ala Ala Ala Ala Ala Ala Lys Ala Ala Lys Tyr Gly Ala Ala Ala
   305                 310                 315                 320
   Gly Leu Val Pro Gly Gly Pro Gly Phe Gly Pro Gly Val Val Gly Val
                   325                 330                 335
   Pro Gly Ala Gly Val Pro Gly Val Gly Val Pro Gly Ala Gly Ile Pro
               340                 345                 350
   Val Val Pro Gly Ala Gly Ile Pro Gly Ala Ala Val Pro Gly Val Val
           355                 360                 365
   Ser Pro Glu Ala Ala Ala Lys Ala Ala Ala Lys Ala Ala Lys Tyr Gly
       370                 375                 380
   Ala Arg Pro Gly Val Gly Val Gly Gly Ile Pro Thr Tyr Gly Val Gly
   385                 390                 395                 400
   Ala Gly Gly Phe Pro Gly Phe Gly Val Gly Val Gly Gly Ile Pro Gly
                   405                 410                 415
   Val Ala Gly Val Pro Gly Val Gly Gly Val Pro Gly Val Gly Gly Val
               420                 425                 430
   Pro Gly Val Gly Ile Ser Pro Glu Ala Gln Ala Ala Ala Ala Ala Lys
           435                 440                 445
   Ala Ala Lys Tyr Gly Val Gly Thr Pro Ala Ala Ala Ala Ala Lys Ala
       450                 455                 460
   Ala Ala Lys Ala Ala Gln Phe Gly Leu Val Pro Gly Val Gly Val Ala
   465                 470                 475                 480
   Pro Gly Val Gly Val Ala Pro Gly Val Gly Val Ala Pro Gly Val Gly
                   485                 490                 495
   Leu Ala Pro Gly Val Gly Val Ala Pro Gly Val Gly Val Ala Pro Gly
   500 505 510
   Val Gly Val Ala Pro Gly Ile Gly Pro Gly Gly Val Ala Ala Ala Ala
   515 520 525
   Lys Ser Ala Ala Lys Val Ala Ala Lys Ala Gln Leu Arg Ala Ala Ala
   530 535 540
   Gly Leu Gly Ala Gly Ile Pro Gly Leu Gly Val Gly Val Gly Val Pro
   545 550 555 560
   Gly Leu Gly Val Gly Ala Gly Val Pro Gly Leu Gly Val Gly Ala Gly
   565 570 575
   Val Pro Gly Phe Gly Ala Val Pro Gly Ala Leu Ala Ala Ala Lys Ala
   580 585 590
   Ala Lys Tyr Gly Ala Ala Val Pro Gly Val Leu Gly Gly Leu Gly Ala
   595 600 605
   Leu Gly Gly Val Gly Ile Pro Gly Gly Val Val Gly Ala Gly Pro Ala
   610 615 620
   Ala Ala Ala Ala Ala Ala Lys Ala Ala Ala Lys Ala Ala Gln Phe Gly
   625 630 635 640
   Leu Val Gly Ala Ala Gly Leu Gly Gly Leu Gly Val Gly Gly Leu Gly
   645 650 655
   Val Pro Gly Val Gly Gly Leu Gly Gly Ile Pro Pro Ala Ala Ala Ala
   660 665 670
   Lys Ala Ala Lys Tyr Gly Ala Ala Gly Leu Gly Gly Val Leu Gly Gly
   675 680 685
   Ala Gly Gln Phe Pro Leu Gly Gly Val Ala Ala Arg Pro Gly Phe Gly
   690 695 700
   Leu Ser Pro Ile Phe Pro Gly Gly Ala Cys Leu Gly Lys Ala Cys Gly
   705 710 715 720
   Arg Lys Arg Lys
<210> 8
   <211> 677
   <212> PRT
   <213> Homo sapiens
<220>
   <223> Sonstige Angaben
<400> 8
   Met Ala Gly Leu Thr Ala Ala Ala Pro Arg Pro Gly Val Leu Leu Leu
   1               5                   10                  15
   Leu Leu Ser Ile Leu His Pro Ser Arg Pro Gly Gly Val Pro Gly Ala
               20                  25                  30
   Ile Pro Gly Gly Val Pro Gly Gly Val Phe Tyr Pro Ala Leu Gly Pro
           35                  40                  45
   Gly Gly Lys Pro Leu Lys Pro Val Pro Gly Gly Leu Ala Gly Ala Gly
       50                  55                  60
   Leu Gly Ala Gly Leu Gly Ala Phe Pro Ala Val Thr Phe Pro Gly Ala
   65                  70                  75                  80
   Leu Val Pro Gly Gly Val Ala Asp Ala Ala Ala Ala Tyr Lys Ala Ala
                   85                  90                  95
   Lys Ala Gly Ala Gly Leu Gly Gly Val Pro Gly Val Gly Gly Leu Gly
               100                 105                 110
   Val Ser Ala Gly Ala Val Val Pro Gln Pro Gly Ala Gly Val Lys Pro
           115                 120                 125
   Gly Lys Val Pro Gly Val Gly Leu Pro Gly Val Tyr Pro Gly Gly Val
       130                 135                 140
   Leu Pro Gly Ala Arg Phe Pro Gly Val Gly Val Leu Pro Gly Val Pro
   145                 150                 155                 160
   Thr Gly Ala Gly Val Lys Pro Lys Ala Pro Gly Val Gly Gly Ala Phe
                   165                 170                 175
   Ala Gly Ile Pro Gly Val Gly Pro Phe Gly Gly Pro Gln Pro Gly Val
               180                 185                 190
   Pro Leu Gly Tyr Pro Ile Lys Ala Pro Lys Leu Pro Gly Gly Tyr Gly
           195                 200                 205
   Leu Pro Tyr Thr Thr Gly Lys Leu Pro Tyr Gly Tyr Gly Pro Gly Gly
       210                 215                 220
   Val Ala Gly Ala Ala Gly Lys Ala Gly Tyr Pro Thr Gly Thr Gly Val
   225                 230                 235                 240
   Gly Pro Gln Ala Ala Ala Ala Ala Ala Ala Lys Ala Ala Ala Lys Phe
                   245                 250                 255
   Gly Ala Gly Ala Ala Gly Val Leu Pro Gly Val Gly Gly Ala Gly Val
               260                 265                 270
   Pro Gly Val Pro Gly Ala Ile Pro Gly Ile Gly Gly Ile Ala Gly Val
           275                 280                 285
   Gly Thr Pro Ala Ala Ala Ala Ala Ala Ala Ala Ala Ala Lys Ala Ala
       290                 295                 300
   Lys Tyr Gly Ala Ala Ala Gly Leu Val Pro Gly Gly Pro Gly Phe Gly
   305                 310                 315                 320
   Pro Gly Val Val Gly Val Pro Gly Ala Gly Val Pro Gly Val Gly Val
                   325                 330                 335
   Pro Gly Ala Gly Ile Pro Val Val Pro Gly Ala Gly Ile Pro Gly Ala
               340                 345                 350
   Ala Val Pro Gly Val Val Ser Pro Glu Ala Ala Ala Lys Ala Ala Ala
           355                 360                 365
   Lys Ala Ala Lys Tyr Gly Ala Arg Pro Gly Val Gly Val Gly Gly Ile
       370                 375                 380
   Pro Thr Tyr Gly Val Gly Ala Gly Gly Phe Pro Gly Phe Gly Val Gly
   385                 390                 395                 400
   Val Gly Gly Ile Pro Gly Val Ala Gly Val Pro Gly Val Gly Gly Val
                   405                 410                 415
   Pro Gly Val Gly Gly Val Pro Gly Val Gly Ile Ser Pro Glu Ala Gln
               420                 425                 430
   Ala Ala Ala Ala Ala Lys Ala Ala Lys Tyr Gly Leu Val Pro Gly Val
           435                 440                 445
   Gly Val Ala Pro Gly Val Gly Val Ala Pro Gly Val Gly Val Ala Pro
       450                 455                 460
   Gly Val Gly Leu Ala Pro Gly Val Gly Val Ala Pro Gly Val Gly Val
   465                 470                 475                 480
   Ala Pro Gly Val Gly Val Ala Pro Gly Ile Gly Pro Gly Gly Val Ala
   485 490 495
   Ala Ala Ala Lys Ser Ala Ala Lys Val Ala Ala Lys Ala Gln Leu Arg
   500 505 510
   Ala Ala Ala Gly Leu Gly Ala Gly Ile Pro Gly Leu Gly Val Gly Val
   515 520 525
   Gly Val Pro Gly Leu Gly Val Gly Ala Gly Val Pro Gly Leu Gly Val
   530 535 540
   Gly Ala Gly Val Pro Gly Phe Gly Ala Val Pro Gly Ala Leu Ala Ala
   545 550 555 560
   Ala Lys Ala Ala Lys Tyr Gly Ala Ala Val Pro Gly Val Leu Gly Gly
   565 570 575
   Leu Gly Ala Leu Gly Gly Val Gly Ile Pro Gly Gly Val Val Gly Ala
   580 585 590
   Gly Pro Ala Ala Ala Ala Ala Ala Ala Lys Ala Ala Ala Lys Ala Ala
   595 600 605
   Gln Phe Gly Leu Val Gly Ala Ala Gly Leu Gly Gly Leu Gly Val Gly
   610 615 620
   Gly Leu Gly Val Pro Gly Val Gly Gly Leu Gly Gly Ile Pro Pro Ala
   625 630 635 640
   Ala Ala Ala Lys Ala Ala Lys Tyr Gly Val Ala Ala Arg Pro Gly Phe
   645 650 655
   Gly Leu Ser Pro Ile Phe Pro Gly Gly Ala Cys Leu Gly Lys Ala Cys
   660 665 670
   Gly Arg Lys Arg Lys
   675
<210> 9
   <211> 692
   <212> PRT
   <213> Homo sapiens
<220>
   <223> Sonstige Angaben
<400> 9
   Met Ala Gly Leu Thr Ala Ala Ala Pro Arg Pro Gly Val Leu Leu Leu
   1               5                   10                  15
   Leu Leu Ser Ile Leu His Pro Ser Arg Pro Gly Gly Val Pro Gly Ala
               20                  25                  30
   Ile Pro Gly Gly Val Pro Gly Gly Val Phe Tyr Pro Gly Ala Gly Leu
           35                  40                  45
   Gly Ala Leu Gly Gly Gly Ala Leu Gly Pro Gly Gly Lys Pro Leu Lys
       50                  55                  60
   Pro Val Pro Gly Gly Leu Ala Gly Ala Gly Leu Gly Ala Gly Leu Gly
   65                  70                  75                  80
   Ala Phe Pro Ala Val Thr Phe Pro Gly Ala Leu Val Pro Gly Gly Val
                   85                  90                  95
   Ala Asp Ala Ala Ala Ala Tyr Lys Ala Ala Lys Ala Gly Ala Gly Leu
               100                 105                 110
   Gly Gly Val Pro Gly Val Gly Gly Leu Gly Val Ser Ala Ala Pro Ser
           115                 120                 125
   Val Pro Gly Ala Val Val Pro Gln Pro Gly Ala Gly Val Lys Pro Gly
       130                 135                 140
   Lys Val Pro Gly Val Gly Leu Pro Gly Val Tyr Pro Gly Gly Val Leu
   145                 150                 155                 160
   Pro Gly Ala Arg Phe Pro Gly Val Gly Val Leu Pro Gly Val Pro Thr
                   165                 170                 175
   Gly Ala Gly Val Lys Pro Lys Ala Pro Gly Val Gly Gly Ala Phe Ala
               180                 185                 190
   Gly Ile Pro Gly Val Gly Pro Phe Gly Gly Pro Gln Pro Gly Val Pro
           195                 200                 205
   Leu Gly Tyr Pro Ile Lys Ala Pro Lys Leu Pro Gly Gly Tyr Gly Leu
       210                 215                 220
   Pro Tyr Thr Thr Gly Lys Leu Pro Tyr Gly Tyr Gly Pro Gly Gly Val
   225                 230                 235                 240
   Ala Gly Ala Ala Gly Lys Ala Gly Tyr Pro Thr Gly Thr Gly Val Gly
                   245                 250                 255
   Pro Gln Ala Ala Ala Ala Ala Ala Ala Lys Ala Ala Ala Lys Phe Gly
               260                 265                 270
   Ala Gly Ala Ala Gly Val Leu Pro Gly Val Gly Gly Ala Gly Val Pro
               275                 280                 285
   Gly Val Pro Gly Ala Ile Pro Gly Ile Gly Gly Ile Ala Gly Val Gly
       290                 295                 300
   Thr Pro Ala Ala Ala Ala Ala Ala Ala Ala Ala Ala Lys Ala Ala Lys
   305                 310                 315                 320
   Tyr Gly Ala Ala Ala Gly Leu Val Pro Gly Gly Pro Gly Phe Gly Pro
                   325                 330                 335
   Gly Val Val Gly Val Pro Gly Ala Gly Val Pro Gly Val Gly Val Pro
               340                 345                 350
   Gly Ala Gly Ile Pro Val Val Pro Gly Ala Gly Ile Pro Gly Ala Ala
           355                 360                 365
   Val Pro Gly Val Val Ser Pro Glu Ala Ala Ala Lys Ala Ala Ala Lys
       370                 375                 380
   Ala Ala Lys Tyr Gly Ala Arg Pro Gly Val Gly Val Gly Gly Ile Pro
   385                 390                 395                 400
   Thr Tyr Gly Val Gly Ala Gly Gly Phe Pro Gly Phe Gly Val Gly Val
                   405                 410                 415
   Gly Gly Ile Pro Gly Val Ala Gly Val Pro Gly Val Gly Gly Val Pro
               420                 425                 430
   Gly Val Gly Gly Val Pro Gly Val Gly Ile Ser Pro Glu Ala Gln Ala
           435                 440                 445
   Ala Ala Ala Ala Lys Ala Ala Lys Tyr Gly Leu Val Pro Gly Val Gly
       450                 455                 460
   Val Ala Pro Gly Val Gly Val Ala Pro Gly Val Gly Val Ala Pro Gly
   465                 470                 475                 480
   Val Gly Leu Ala Pro Gly Val Gly Val Ala Pro Gly Val Gly Val Ala
                   485                 490                 495
   Pro Gly Val Gly Val Ala Pro Gly Ile Gly Pro Gly Gly Val Ala Ala
               500                 505                 510
   Ala Ala Lys Ser Ala Ala Lys Val Ala Ala Lys Ala Gln Leu Arg Ala
                       515                 520                 525
   Ala Ala Gly Leu Gly Ala Gly Ile Pro Gly Leu Gly Val Gly Val Gly
       530                 535                 540
   Val Pro Gly Leu Gly Val Gly Ala Gly Val Pro Gly Leu Gly Val Gly
   545                 550                 555                 560
   Ala Gly Val Pro Gly Phe Gly Ala Val Pro Gly Ala Leu Ala Ala Ala
                   565                 570                 575
   Lys Ala Ala Lys Tyr Gly Ala Ala Val Pro Gly Val Leu Gly Gly Leu
               580                 585                 590
   Gly Ala Leu Gly Gly Val Gly Ile Pro Gly Gly Val Val Gly Ala Gly
           595                 600                 605
   Pro Ala Ala Ala Ala Ala Ala Ala Lys Ala Ala Ala Lys Ala Ala Gln
       610                 615                 620
   Phe Gly Leu Val Gly Ala Ala Gly Leu Gly Gly Leu Gly Val Gly Gly
   625                 630                 635                 640
   Leu Gly Val Pro Gly Val Gly Gly Leu Gly Gly Ile Pro Pro Ala Ala
                   645                 650                 655
   Ala Ala Lys Ala Ala Lys Tyr Gly Val Ala Ala Arg Pro Gly Phe Gly
               660                 665                 670
   Leu Ser Pro Ile Phe Pro Gly Gly Ala Cys Leu Gly Lys Ala Cys Gly
           675                 680                 685
   Arg Lys Arg Lys
       690
<210> 10
   <211> 711
   <212> PRT
   <213> Homo sapiens
<220>
   <223> Sonstige Angaben
<400> 10
   Met Ala Gly Leu Thr Ala Ala Ala Pro Arg Pro Gly Val Leu Leu Leu
   1               5                   10                  15
   Leu Leu Ser Ile Leu His Pro Ser Arg Pro Gly Gly Val Pro Gly Ala
               20                  25                  30
   Ile Pro Gly Gly Val Pro Gly Gly Val Phe Tyr Pro Gly Ala Gly Leu
           35                  40                  45
   Gly Ala Leu Gly Gly Gly Ala Leu Gly Pro Gly Gly Lys Pro Leu Lys
       50                  55                  60
   Pro Val Pro Gly Gly Leu Ala Gly Ala Gly Leu Gly Ala Gly Leu Gly
   65                  70                  75                  80
   Ala Phe Pro Ala Val Thr Phe Pro Gly Ala Leu Val Pro Gly Gly Val
                   85                  90                  95
   Ala Asp Ala Ala Ala Ala Tyr Lys Ala Ala Lys Ala Gly Ala Gly Leu
               100                 105                 110
   Gly Gly Val Pro Gly Val Gly Gly Leu Gly Val Ser Ala Ala Pro Ser
           115                 120                 125
   Val Pro Gly Ala Val Val Pro Gln Pro Gly Ala Gly Val Lys Pro Gly
       130                 135                 140
   Lys Val Pro Gly Val Gly Leu Pro Gly Val Tyr Pro Gly Gly Val Leu
   145                 150                 155                 160
   Pro Gly Ala Arg Phe Pro Gly Val Gly Val Leu Pro Gly Val Pro Thr
                   165                 170                 175
   Gly Ala Gly Val Lys Pro Lys Ala Pro Gly Val Gly Gly Ala Phe Ala
               180                 185                 190
   Gly Ile Pro Gly Val Gly Pro Phe Gly Gly Pro Gln Pro Gly Val Pro
           195                 200                 205
   Leu Gly Tyr Pro Ile Lys Ala Pro Lys Leu Pro Gly Gly Tyr Gly Leu
       210                 215                 220
   Pro Tyr Thr Thr Gly Lys Leu Pro Tyr Gly Tyr Gly Pro Gly Gly Val
   225                 230                 235                 240
   Ala Gly Ala Ala Gly Lys Ala Gly Tyr Pro Thr Gly Thr Gly Val Gly
                   245                 250                 255
   Pro Gln Ala Ala Ala Ala Ala Ala Ala Lys Ala Ala Ala Lys Phe Gly
               260                 265                 270
   Ala Gly Ala Ala Gly Val Leu Pro Gly Val Gly Gly Ala Gly Val Pro
           275                 280                 285
   Gly Val Pro Gly Ala Ile Pro Gly Ile Gly Gly Ile Ala Gly Val Gly
   290 295 300
   Thr Pro Ala Ala Ala Ala Ala Ala Ala Ala Ala Ala Lys Ala Ala Lys
   305 310 315 320
   Tyr Gly Ala Ala Ala Gly Leu Val Pro Gly Gly Pro Gly Phe Gly Pro
   325 330 335
   Gly Val Val Gly Val Pro Gly Ala Gly Val Pro Gly Val Gly Val Pro
   340 345 350
   Gly Ala Gly Ile Pro Val Val Pro Gly Ala Gly Ile Pro Gly Ala Ala
   355 360 365
   Val Pro Gly Val Val Ser Pro Glu Ala Ala Ala Lys Ala Ala Ala Lys
   370 375 380
   Ala Ala Lys Tyr Gly Ala Arg Pro Gly Val Gly Val Gly Gly Ile Pro
   385 390 395 400
   Thr Tyr Gly Val Gly Ala Gly Gly Phe Pro Gly Phe Gly Val Gly Val
   405 410 415
   Gly Gly Ile Pro Gly Val Ala Gly Val Pro Gly Val Gly Gly Val Pro
   420 425 430
   Gly Val Gly Gly Val Pro Gly Val Gly Ile Ser Pro Glu Ala Gln Ala
   435 440 445
   Ala Ala Ala Ala Lys Ala Ala Lys Tyr Gly Val Gly Thr Pro Ala Ala
   450 455 460
   Ala Ala Ala Lys Ala Ala Ala Lys Ala Ala Gln Phe Gly Leu Val Pro
   465 470 475 480
   Gly Val Gly Val Ala Pro Gly Val Gly Val Ala Pro Gly Val Gly Val
   485 490 495
   Ala Pro Gly Val Gly Leu Ala Pro Gly Val Gly Val Ala Pro Gly Val
   500 505 510
   Gly Val Ala Pro Gly Val Gly Val Ala Pro Gly Ile Gly Pro Gly Gly
   515 520 525
   Val Ala Ala Ala Ala Lys Ser Ala Ala Lys Val Ala Ala Lys Ala Gln
       530                 535                 540
   Leu Arg Ala Ala Ala Gly Leu Gly Ala Gly Ile Pro Gly Leu Gly Val
   545                 550                 555                 560
   Gly Val Gly Val Pro Gly Leu Gly Val Gly Ala Gly Val Pro Gly Leu
                   565                 570                 575
   Gly Val Gly Ala Gly Val Pro Gly Phe Gly Ala Val Pro Gly Ala Leu
               580                 585                 590
   Ala Ala Ala Lys Ala Ala Lys Tyr Gly Ala Ala Val Pro Gly Val Leu
           595                 600                 605
   Gly Gly Leu Gly Ala Leu Gly Gly Val Gly Ile Pro Gly Gly Val Val
       610                 615                 620
   Gly Ala Gly Pro Ala Ala Ala Ala Ala Ala Ala Lys Ala Ala Ala Lys
   625                 630                 635                 640
   Ala Ala Gln Phe Gly Leu Val Gly Ala Ala Gly Leu Gly Gly Leu Gly
                   645                 650                 655
   Val Gly Gly Leu Gly Val Pro Gly Val Gly Gly Leu Gly Gly Ile Pro
               660                 665                 670
   Pro Ala Ala Ala Ala Lys Ala Ala Lys Tyr Gly Val Ala Ala Arg Pro
           675                 680                 685
   Gly Phe Gly Leu Ser Pro Ile Phe Pro Gly Gly Ala Cys Leu Gly Lys
       690                 695                 700
   Ala Cys Gly Arg Lys Arg Lys
   705                 710
<210> 11
   <211> 705
   <212> PRT
   <213> Homo sapiens
<220>
   <223> Sonstige Angaben
<400> 11